(19) Europäisches Patentamt
European Patent Office
Office européen des brevets

(11) EP 0 549 967 B1

(12) **EUROPÄISCHE PATENTSCHRIFT**

(45) Veröffentlichungstag und Bekanntmachung des Hinweises auf die Patenterteilung:
**13.03.1996 Patentblatt 1996/11**

(51) Int Cl.[6]: **C08F 20/54**, C08F 12/26, A61K 31/785, C07J 9/00, C07J 41/00

(21) Anmeldenummer: **92121459.9**

(22) Anmeldetag: **17.12.1992**

(54) **Polymere und Oligomere von Gallensäurederivaten, Verfahren zu ihrer Herstellung und ihre Verwendung als Arzneimittel**

Polymers and oligomers of bile-acids, process for their production and their use as medicines

Polymères et oligomères des acides billiaires, procédé pour leur préparation et leur utilisation comme médicaments

(84) Benannte Vertragsstaaten:
**AT BE CH DE DK ES FR GB GR IE IT LI LU MC NL PT SE**

(30) Priorität: **20.12.1991 DE 4142379**

(43) Veröffentlichungstag der Anmeldung:
**07.07.1993 Patentblatt 1993/27**

(73) Patentinhaber: **HOECHST AKTIENGESELLSCHAFT**
**D-65926 Frankfurt am Main (DE)**

(72) Erfinder:
- **Kramer, Werner, Dr. Dr.**
  **W-6500 Mainz 43 (DE)**
- **Müllner, Stefan, Dr.**
  **W-6203 Hochheim (DE)**
- **Gutweiler, Matthias, Dr.**
  **W-6204 Taunusstein (DE)**
- **Kroggel, Matthias, Dr.**
  **W-6233 Kelkheim/Ts. (DE)**

(56) Entgegenhaltungen:
**US-A- 4 104 285**

- **CHEMICAL ABSTRACTS, vol. 85, no. 26, 27. Dezember 1976, Columbus, Ohio, US; abstract no. 193309, 'POLYMERS BY RADICAL POLYMERIZATION OF METHACRYLIC COMPOUNDS CONTAINING CHOLESTEROL IN THE SIDE CHAIN' Seite 13 ;Spalte 1 ;**

EP 0 549 967 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft Polymere und Oligomere von Gallensäuren und deren Copolymere, ein Verfahren zu deren Herstellung sowie ihre Verwendung als Arzneimittel.

Gallensäuren bzw. deren Salze sind natürliche Detergentien und haben eine wichtige physiologische Funktion bei der Fettverdauung, z.B. als Cofaktoren der pankreatischen Lipasen und bei der Fettresorption. Als Endprodukt des Cholesterinstoffwechsels werden sie in der Leber synthetisiert, in der Gallenblase gespeichert und aus dieser durch Kontraktion in den Dünndarm abgegeben, wo sie ihre physiologische Wirkung entfalten. Der größte Teil der sezernierten Gallensäuren wird über den enterohepatischen Kreislauf wieder zurückgewonnen. Sie gelangen über die Mesenterialvenen des Dünndarms und das Pfortadersystem wieder zur Leber zurück.

Bei der Rückresorption im Darm spielen sowohl aktive als auch passive Transportprozesse eine Rolle. Im terminalen Ileum ist ein spezifisches $Na^+$-abhängiges Transportsystem für die Gallensäurerückresorption verantwortlich. Im enterohepatischen Kreislauf treten die Gallensäuren sowohl als freie Säuren, aber auch in Form von Aminosäurekonjugaten, wie Glycin- und Taurinkonjugaten, in Erscheinung.

Nicht resorbierbare, unlösliche, basische und vernetzte Polymere (Resins) werden seit geraumer Zeit zur Bindung von Gallensäuren verwendet und aufgrund dieser Eigenschaften therapeutisch genutzt. Als Therapieobjekt werden alle Erkrankungen, bei denen eine Hemmung der Gallensäurerückresorption im Darm, insbesondere im Dünndarm, wünschenswert erscheint, angesehen. Beispielsweise werden die chologene Diarrhoe nach Ileumresektion oder auch erhöhte Cholesterin-Blutspiegel auf diese Weise behandelt. Im Falle des erhöhten Cholesterin-Blutspiegels kann durch den Eingriff in den enterohepatischen Kreislauf eine Senkung dieses Spiegels erreicht werden.

Durch Senkung des im enterohepatischen Kreislauf befindlichen Gallensäurepools wird die entsprechende Neusynthese von Gallensäuren aus Cholesterin in der Leber erzwungen. Zur Deckung des Cholesterinbedarfs in der Leber, wird dann auf das im Blutkreislauf befindliche LDL-Cholesterin (Low Density Lipoprotein) zurückgegriffen, wobei die hepatischen LDL-Rezeptoren in vermehrter Anzahl zur Wirkung kommen. Die so erfolgte Beschleunigung des LDL-Katabolismus wirkt sich durch die Herabsetzung des atherogenen Cholesterinanteils im Blut aus.

Bislang stellten die polymeren, basischen unlöslichen und vernetzten Ionenaustauscher-Harze (Resins) die einzige Möglichkeit dar, den enterohepatischen Kreislauf hinsichtlich erhöhter Gallensäureausscheidung und daraus folgender Senkung des Cholesterinspiegels zu beeinflussen (US-A-3,383,281).

Es war deshalb die Aufgabe der vorliegenden Erfindung, nach weiteren Möglichkeiten zu suchen, Einfluß auf den enterohepatischen Kreislauf im Hinblick auf erhöhte Gallensäureausscheidung zu nehmen, ohne die Nachteile der bisher eingesetzten Resins weiterzuführen.

Die Aufgabe wird gelöst durch Bereitstellung von polymeren oder oligomeren Gallensäuren, herstellbar durch Polymerisation von monomeren Gallensäuren der Formel I

$$G - X - A \qquad (I)$$

in der

G ein Gallensäurerest oder -derivat,
X eine Brückengruppe und
A eine polymersierbare, ethylenisch ungesättigte Gruppe bedeutet,
oder durch Copolymerisation mit einem, eine polymerisierbare, ethylenisch ungesättigte Doppelbindung aufweisenden Monomeren, insbesondere durch Copolymerisation mit einem Monomeren der Formel IV

$$H_2C = C(R^9) - R^{10}, \qquad IV$$

worin

$R^9$ Wasserstoff oder Methyl und
$R^{10}$

$$-\overset{\displaystyle O}{\overset{\|}{C}}-O-R^{11},\ -\overset{\displaystyle O}{\overset{\|}{C}}-NR^{12}R^{13},\ -O-\overset{\displaystyle O}{\overset{\|}{C}}-R^{14},$$

-CN, -O-$R^{15}$, Wasserstoff, Halogen, insbesondere Chlor, Brom oder Iod, -$SO_3H$ oder -O-($CH_2$-$CH_2O$)$_n$$R^{16}$, worin

$R^{11}$ Wasserstoff, ($C_1$-$C_{10}$)-Alkyl, ($C_1$-$C_{10}$)-mono-Hydroxyalkyl oder -($CH_2CH_2$-O-)$_n$$R^{16}$,

$R^{12}$, $R^{13}$, $R^{15}$ und $R^{16}$ gleich oder verschieden sind und ($C_1$-$C_{10}$)-Alkyl,

$R^{14}$ ($C_1$-$C_{18}$)-Alkyl und

n 1 bis 50

bedeuten,

oder durch Copolymerisation mit N-Vinylpyrrolidon oder dessen Derivaten,
und/oder durch Copolymerisation mit ethylenisch ungesättigten Dicarbonsäureanhydriden und ethylenisch ungesättigten Dicarbonsäuren mit jeweils 2 bis 6 C-Atomen; deren Estern oder Halbestern, wobei als Ester Alkylester mit 1 - 6 C-Atomen, Cycloalkylester mit 5 bis 8 C-Atomen, Benzyl- oder Phenylester zu verstehen sind.

Oligomere steht sowohl für Homo-oligomere als auch für Co-oligomere. Polymere steht sowohl für Homo-polymere als auch für Co-polymere.

Die erfindungsgemäßen Verbindungen sind vernetzt oder unvernetzt. Polymerisation und Copolymerisation stehen ebenfalls für Oligomerisation und Cooligomerisation.

Unter den Verbindungen der Formel I sind bevorzugt folgende:

Verbindungen, in denen

G eine freie Gallensäure bzw. ihr Alkali- oder Erdalkalisalz oder eine am Ring D veresterte Gallensäure, die über ihren Ring A oder B, vorzugsweise über Ring A, verbunden ist mit der Gruppe

X, für die bevorzugt die Formel II gilt

$$(Y)_o - (Z)_p \qquad (II),$$

worin

Y benachbart zu G steht und -O-, -NR'-,

$$-O-\overset{\displaystyle O}{\overset{\|}{C}}-,\ -NR'-\overset{\displaystyle O}{\overset{\|}{C}}-,$$

Z ($C_1$-$C_{12}$)-Alkylen oder ($C_7$-$C_{13}$)-Aralkylen, wobei einzelne, bevorzugt 1 bis 4, Methylengruppen in der Alkylenkette des Alkylen- oder Aralkylenrestes durch Gruppen wie -O-, -NR'-,

$$-O-\overset{\displaystyle O}{\overset{\|}{C}}-,\ -NR'-\overset{\displaystyle O}{\overset{\|}{C}}-,$$

oder

$$-NR'-\underset{\displaystyle O}{\underset{\|}{C}}-NR''-,$$

bevorzugt eine Gruppe eines Types, ersetzt sein können,

o und p unabhängig voneinander null oder 1, wobei o und p nicht gleichzeitig null sind,

A eine ethylenisch ungesättigte Gruppe der Formel

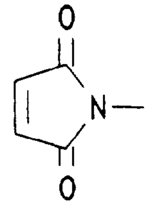

oder 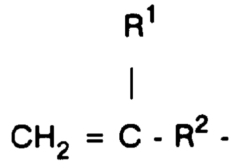

bedeuten, worin

$R^1$ Wasserstoff oder $CH_3$ und

$R^2$

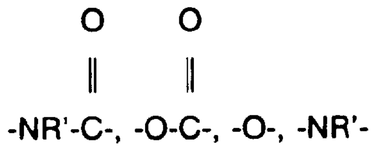

oder eine Einfachbindung bedeutet, wobei die Carbonylgruppen benachbart zur C-C-Doppelbindung stehen,

R' und R" unabhängig voneinander Wasserstoff oder $(C_1\text{-}C_6)$-Alkyl, bevorzugt $(C_1\text{-}C_3)$-Alkyl bedeuten.

Hierunter bevorzugt sind Polymere und Oligomere, worin G der Formel III

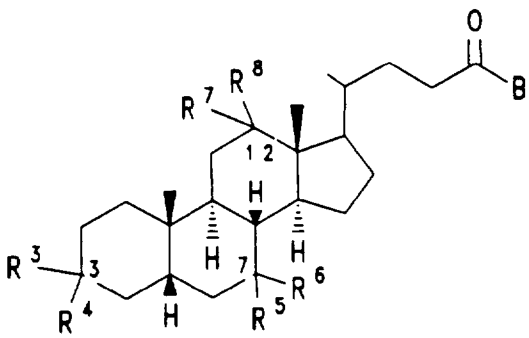

(III)

entspricht, worin

$R^3$ bis $R^8$ unabhängig voneinander Wasserstoff, OH, $NH_2$ oder eine mit einer OH-Schutzgruppe geschützte OH-Gruppe und einer der Reste $R^3$ bis $R^6$ eine Bindung zur Gruppe X bedeuten, wobei diese Bindung von den Positionen 3 ($R^3$ oder $R^4$) oder 7 ($R^5$ oder $R^6$), bevorzugt ist die β-Position, ausgeht und die jeweils andere Position 7 oder 3 eine OH-Gruppe oder eine geschützte OH-Gruppe trägt,

B -OH, -O-Alkali, -O-Erdalkali, -O-$(C_1\text{-}C_{12})$-Alkyl, -O-Allyl oder -O-Benzyl bedeutet, bevorzugt -OH, -O-Alkali, -O-$(C_1\text{-}C_6)$-Alkyl, -O-Allyl oder -O-Benzyl, wobei Alkyl sowohl n-Alkyl wie iso-Alkyl bedeutet und wobei die gebildete Estergruppe

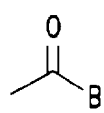

sowohl sauer wie auch basisch verseifbare Ester darstellt,

Y

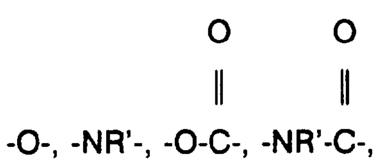

Z $(C_1\text{-}C_{12})$-Alkylen, $(C_7\text{-}C_{13})$-Aralkylen, wobei 1 bis 3 Methylengruppen in der Alkylenkette durch die Gruppen

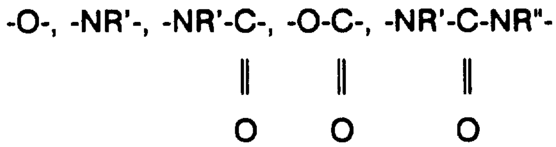

ersetzt sind und

o und p unabhängig voneinander null oder 1 bedeuten, wobei o und p nicht gleichzeitig null sind,

A

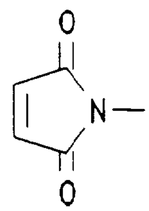

oder 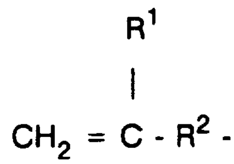

bedeuten, wobei

$R^1$ Wasserstoff oder $CH_3$ und

$R^2$

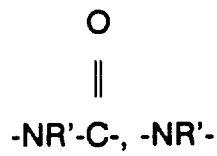

oder eine Einfachbindung bedeutet, worin

R' und R" unabhängig voneinander Wasserstoff oder $(C_1-C_6)$-Alkyl bedeuten.

Sofern p = null und o = 1 gilt, ist Y bevorzugt

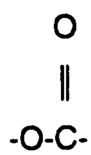 oder 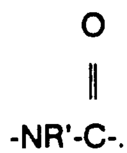

Sofern p = 1 und o = null gilt, ist Z bevorzugt $(C_1-C_{12})$-Alkylen, wobei 1 - 3 Methylengruppen, bevorzugt eine Methylengruppe, durch

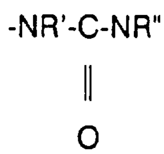

ersetzt sind.

Sofern p = 1 und o = 1 gilt, ist Y bevorzugt -O-. Hierunter ist bevorzugt, daß Z $(C_1-C_{12})$-Alkylen oder $(C_7-C_{13})$-Aralkylen bedeutet, wobei 1 oder 2 Methylengruppen, bevorzugt eine Methylengruppe, durch

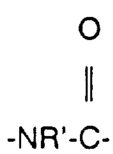 oder 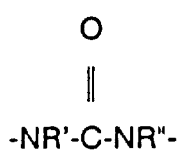

ersetzt sind.

Weiterhin ist hierunter bevorzugt, daß eine Methylengruppe von Z dann

$$-NR'-\overset{\overset{O}{\|}}{C}-NR''-$$

bedeutet, wenn Z selbst ein Aralkylrest bedeutet, worin der Arylrest meta-verknüpft ist, Z einerseits als Rest A eine Gruppe

$$CH_2=\overset{\overset{R^1}{|}}{C}-R^2-$$

trägt, worin $R^2$ eine Einfachbindung bedeutet und andererseits eine

$$-NR'-\overset{\overset{O}{\|}}{C}-NR''-$$

Gruppe trägt, die über eine Methylengruppe mit den Aralkylenrest meta-verknüpft ist.

Ebenso ist hierunter bevorzugt, daß, sofern Z eine ($C_1$-$C_{12}$)-Alkylengruppe bedeutet, maximal eine Methylengruppe durch

$$-NR'-\overset{\overset{O}{\|}}{C}-$$

ersetzt ist und als Rest A

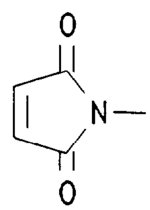

oder $CH_2 = \overset{\overset{R^1}{|}}{C} - R^2 -$ gilt, wobei $R^2$ $-NR'-\overset{\overset{O}{\|}}{C}-$

bedeutet.

Besonders bevorzugt ist weiterhin, daß Y nicht direkt mit der eine Methylengruppe von Z ersetzenden Gruppe direkt benachbart ist und auch nicht mit

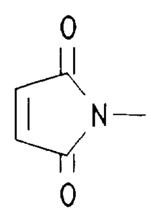

oder $CH_2 = \overset{\overset{R^1}{|}}{C} - R^2 -$

benachbart ist, sofern $R^2$ eine Einfachbindung bedeutet.

Die Verbindungen der Formel I sind Gegenstand von EP-0548793.

Unter den OH-Schutzgruppen werden verstanden

ein Alkylrest mit 1 - 10 C-Atomen oder ein Alkenylrest mit 2 - 10 C-Atomen, wobei die Reste verzweigt oder unverzweigt sind,
ein Cycloalkylrest mit 3-8 C-Atomen,
ein Phenylrest, der unsubstituiert oder 1-3fach substituiert ist mit F, Cl, Br, $(C_1-C_4)$-Alkyl oder $(C_1-C_4)$-Alkoxy,
ein Benzylrest, der unsubstituiert oder 1-3fach substituiert ist mit F, Cl, Br, $(C_1-C_4)$-Alkyl oder $(C_1-C_4)$-Alkoxy oder ein

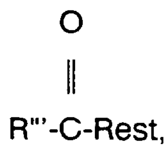

wobei R''' Wasserstoff oder $(C_1-C_4)$-Alkyl bedeutet.

Bevorzugte Comonomere der Formel IV sind:
(Meth)acrylsäure, (Meth)acrylsäureester, Acrylamid, Acrylamid-Derivate. Besonders bevorzugt sind Carbonsäurevinylester mit 3-20 C-Atomen und N-Vinylpyrrolidon sowie dessen Derivate. Die Monomeren werden gegebenenfalls in Mischung eingesetzt.

Die beschriebenen Polymeren oder Copolymeren von Gallensäurederivaten können auch zusätzlich vernetzt sein durch Copolymerisation mit mehrfach ethylenisch ungesättigten Monomeren. Vorzugsweise werden zweifach oder dreifach ethylenisch ungesättigte Acryl- und Methacrylasäurederivate genannt. Erfindungsgemäße polymere und oligomere Gallensäuren können auch durch polymeranaloge Reaktionen mit üblichen bifunktionellen Reagentien vernetzt sein.

Insbesondere kommen als Vernetzer die Säureamide der genannten Verbindungen in Frage und hierunter wiederum Säureamide der allgemeinen Formel V

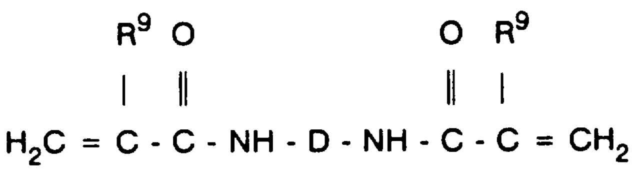

V

worin

$R^9$ Wasserstoff sowie Methyl und
D $-(CHE)_m-$ bedeutet,
wobei
m 1 bis 10, vorzugsweise 2 bis 4 und
E Wasserstoff oder OH, vorzugsweise Wasserstoff für m = 2
bedeutet.

Gegenstand der Erfindung ist weiterhin ein Verfahren zur Herstellung der polymeren Gallensäuren.

Die radikalische Polymerisation bzw. Copolymerisation wird in Suspension oder Emulsion, ggf. in Substanz, bevorzugt aber in Lösung unter Verwendung von Radikalbildnern bei Temperaturen unter 250°C, bevorzugt bei Temperaturen von 40 bis 100°C durchgeführt.

Geeignete Radikalbildner sind anorganische oder organische Peroxide, Percarbonate und Azoverbindungen, die bevorzugt in Mengen von 0,01 bis 30 mol-% bezgl. der von polymerisierenden Monomere eingesetzt werden. Bevorzugt ist die Verwendung von Kaliumperoxodisulfat, Wasserstoffperoxid, Dilaurylperoxid oder besonders bevorzugt wird Azobisisobutyronitril, tert.-Butylperoxydiethylacetat, Dibenzoylperoxid oder tert.-Butyloxy-2-ethylhexanoat.

Weiterhin einsetzbar sind:
tert.-Butylperoxyisobutyrate, tert.-Butylperoxyisopropylcarbonat, tert.-Butylperoxy-3,5,5-trimethylhexanoat, 2,2-Bis (tert.-butyl)peroxybutan, tert.-Butylperoxystearylcarbonate, tert.-Butylperoxyacetate, tert.-Butylperoxybenzoat, Dicumylperoxid, 2,5-Dimethyl-2,5-bis(tert.-butylperoxy)hexan, tert.-Butylcumylperoxid, 1,3-Bis(tert.-butyl)-peroxy-isopropyl) benzol, Di-tert.-butylperoxid, Bis(2-methylbenzoyl)-peroxid, Bis(3,5,5-trimethylhexanoyl)peroxid, tert.-Butylperoxypivalat, tert.-Amylperoxypivalat, tert.-Butylperoxyneodecanoat, tert.-Amylperoxyneodecanoat, Diisopropylperoxydicarbonat, Bis(2-ethylhexyl)peroxydicarbonat, Di-n-butylperoxydicarbonat, Di-sec-butylperoxydicarbonat u.a..

Die Art der eingesetzten Lösungsmittel richtet sich nach der Löslichkeit der eingesetzten Monomeren. Wasserlösliche Monomere werden in wäßriger Lösung polymerisiert. In organischen Lösungsmitteln lösliche Monomere lassen sich prinzipiell in allen organischen Lösungsmitteln polymerisieren, in denen üblicherweise radikalische Polymerisationen durchgeführt werden. Eingesetzt werden beispielsweise Dimethylsulfoxid, Dimethylformamid, Chloroform, Methylenchlorid, Ester mit bis zu 10 C-Atomen, beispielsweise Ethylacetat oder Methylacetat oder Kohlenwasserstoffe wie Benzol, Toluol oder Xylol. Bevorzugt sind Alkohole mit bis zu 6 C-Atomen, beispielsweise Methanol, Ethanol, iso-Propanol, Propanol u.a. sowie Ether wie beispielsweise Tetrahydrofuran oder Dioxan. Die Lösungsmittel können ggf. auch in Mischung oder eventuell in Kombination mit Wasser eingesetzt werden.

Das Molekulargewicht der polymeren Produkte läßt sich durch Art und Menge der eingesethen Lösungsmittel sowie der verwendeten Radikalbildner, durch die Reaktionszeit sowie die Reaktionstemperatur steuern. Darüberhinaus ist eine Molekulargewichtssteuerung durch Verwendung von Reglern, bevorzugt in Mengen von bis zu 2 mol-% bzgl. der eingesetzten Monomeren, wie z.B. Alkyl- und Arylmercaptanen, Aldehyden, Phenolen und Aminen möglich.

Die Synthese der Polymeren kann sowohl nach allgemein bekannten Dosierverfahren als auch durch Batch-Reaktion erfolgen.

Die erfindungsgemäßen polymeren Gallensäuren besitzen gewichtsmittlere Molekulargewichte von bevorzugt bis zu 250 000 g/mol. Besonders bevorzugt sind Produkte mit gewichtsmittleren Molekulargewichten zwischen 2000 und 100 000 g/mol, ganz besonders bevorzugt sind Verbindungen deren Molekulargewicht zwischen 3000 und 60 000 g/mol liegt.

Bei copolymeren Verbindungen sollte das molare Verhältnis von Gallensäureeinheiten zu copolymerisierten Monomereinheiten bevorzugt zwischen 300: 1 und 1 : 300 liegen, besonders bevorzugt sind molare Verhältnisse von 150 : 1 bis 1 : 150.

Polymere Gallensäuren der erfindungsgemäßen Art mit verseifbaren bzw. umesterbaren Einheiten lassen sich in Lösung zu den entsprechenden Verbindungen umestern bzw. verseifen. Geeignete Lösungsmittel sind hier bevorzugt Alkohole mit bis zu 6 C-Atomen, Ether wie Diethylether, Tetrahydrofuran, Dioxan, Halogenkohlenwasserstoffe wie Chloroform, Methylenchlorid, darüberhinaus Dimethylsulfoxid oder Dimethylformamid.

Besonders bevorzugt sind wassermischbare Lösungsmittel, die in Kombination mit Wasser eingesetzt werden. Die Verseifungen werden unter Zusatz von Mineralsäuren wie z.B. Salzsäure, Schwefelsäure, organischen Säuren wie z.B. p-Toluolsulfonsäure durchgeführt. Bevorzugt ist allerdings die Verwendung von Basen wie beispielsweise NaOH, KOH, prim. Aminen, sek. Aminen, tert. Aminen, Alkali-und Erdalkali-Alkoholaten u.a..

Die Verseifungen werden in der Regel bei Temperaturen von 15 bis 100°C durchgeführt.

Die Isolierung und Reinigung der verseiften Polymeren kann durch chromatographische Verfahren wie beispielsweise HPLC oder GPC, durch Dialyse und anschließende Gefriertrocknung oder durch Ausfällung der Produkte aus dem Reaktionsansatz erfolgen.

Die Synthese und Eigenschaften der erfindungsgemäßen polymeren Gallensäuren werden durch die nachfolgenden Beispiele näher erläutert. Die aufgeführten Beispiele schränken den Erfindungsgegenstand in keinster Weise ein, sondern sind als Auswahl des Erfindungsgegenstandes zu verstehen.

In den folgenden Beispielen werden Gallensäuremethylester der allgemeinen Formel VI verwendet.

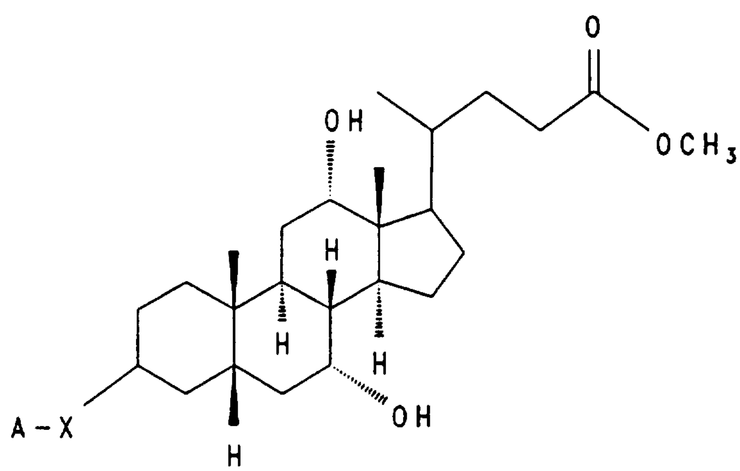

Die Gruppe A-X ist jeweils in den Beispielen definiert.

Für die Dialyse wurde Dialyseschlauch der Firma Spectrum Medical Industries, INC. mit der Bezeichnung Spectra/Por Nr. 3 mit einer Ausschlußgrenze von 3500 g/mol eingesetzt.

Die Bestimmung der gewichtsmittleren Molekulargewichte erfolgte mittels GPC im Vergleich zu Polystyrolstandards.

| | |
|---|---|
| Chromatograph: | ALC/GPC 244 Waters Chromatographie |
| Säulensatz: | 4 Ultrastyragel Säulen |
| Lösemittel: | THF |
| Durchfluß: | 1 ml/min |

Probenmenge: 0,4 ml Probenlösung mit c = 0,2 g/dl
Detektor: RI + 4X

Beispiele

Beispiel 1

In einem Reaktionsgefäß werden unter Stickstoff 1000 mg eines Gallensäuremethylesters, wobei

A-X =
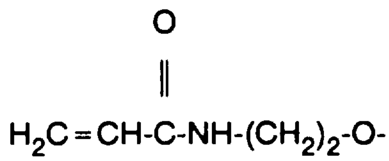

bedeutet, in 4 ml Tetrahydrofuran gelöst und mit 10 g Vinylacetat und 300 mg 75 %igem Dibenzoylperoxid versetzt. Die Reaktionsmischung wird für 6 Stunden unter Rühren auf 80°C erhitzt. Anschließend versetzt man die Reaktionsmischung mit 15 ml THF und engt am Rotationsverdampfer zur Trockene ein. Der Rückstand wird in 25 ml THF aufgenommen und unter Rühren mit 2 ml 10 %iger methanolischer Natronlauge versetzt und für 2 Stunden auf 40°C erwärmt. Anschließend wird die Reaktionsmischung mit 50 ml Wasser verdünnt, 24 Stunden gegen entsalztes Wasser dialysiert (cut off: 3500 g/mol) und gefriergetrocknet.

Molares Verhältnis von Gallensäureeinheiten zu Vinylalkoholeinheiten:

1 : 80 (ermittelt durch NMR),
gewichtsmittleres Molekulargewicht der unverseiften Substanz:
$M_w$ = 34 000 g/mol (ermittelt mit GPC).

Beispiel 2

In einem Reaktionsgefäß werden unter Stickstoff 3000 mg eines Gallensäuremethylesters, wobei

A-X =
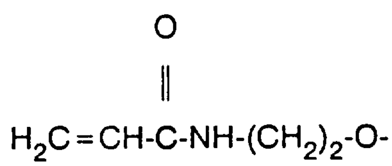

bedeutet, in 4 ml Tetrahydrofuran gelöst und mit 7 g Vinylacetat und 300 mg 75 %igem Dibenzoylperoxid versetzt. Die Reaktionsmischung wird für 6 Stunden unter Rühren auf 80°C erhitzt. Anschließend versetzt man die Reaktionsmischung mit 15 ml THF und engt am Rotationsverdampfer zur Trockene ein. Der Rückstand wird in 25 ml THF aufgenommen und unter Rühren mit 2 ml 10 %iger methanolischer Natronlauge versetzt und für 2 Stunden auf 40°C erwärmt. Anschließend wird die Reaktionsmischung mit 50 ml Wasser verdünnt, 24 Stunden gegen entsalztes Wasser dialysiert (cut off: 3500 g/mol) und gefriergetrocknet.

Molares Verhältnis von Gallensäureeinheiten zu Vinylalkoholeinheiten:

1 : 12,5 (ermittelt durch NMR),
gewichtsmittleres Molekulargewicht der unverseiften Substanz:
$M_w$ = 8 000 g/mol (ermittelt mit GPC).

Beispiel 3

In einem Reaktionsgefäß werden unter Stickstoff 4920 mg eines Gallensäuremethylesters, wobei

A-X
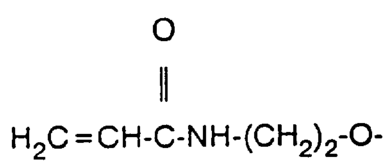

bedeutet, in 4 ml Tetrahydrofuran gelöst und mit 220 mg 75 %igem Dibenzoylperoxid, gelöst in 0,3 ml THF und 0,2 ml Methanol, versetzt. Die Reaktionsmischung wird unter Rühren auf 80°C erhitzt. Zu der Reaktionsmischung werden

innerhalb von 4 Stunden 17,01 g N-Vinylpyrrolidon kontinuierlich zudosiert, wobei nach 2 und nach 4 Stunden Reaktionszeit jeweils 220 mg 75 %iges Dibenzoylperoxid, gelöst in 0,3 ml THF und 0,2 ml Methanol zugesetzt werden. Nach erfolgter Zudosierung des N-Vinylpyrrolidons läßt man für zwei Stunden bei 80°C nachreagieren. Anschließend verdünnt man die Reaktionsmischung mit 15 ml THF und versetzt mit 0,5 ml 20 %iger wäßriger Natronlauge. Nach ca. 10 Minuten wird die auftretende Trübung der Reaktionsmischung durch Zusatz von Wasser in Lösung gebracht. Dieser Vorgang wird so oft wiederholt, bis keine Trübung der Mischung mehr auftritt. Anschließend wird die Reaktionsmischung mit 50 ml Wasser verdünnt, 24 Stunden gegen entsalztes Wasser dialysiert (cut off: 3500 g/mol) und gefriergetrocknet.

Molares Verhältnis von Gallensäureeinheiten zu N-Vinylpyrrolidon-Einheiten:

1 : 1 (ermittelt durch NMR),
gewichtsmittleres Molekulargewicht der unverseiften Substanz:
$M_w$ = 38 000 g/mol (ermittelt mit GPC).

Beispiel 4

In einem Reaktionsgefäß werden unter Stickstoff 3000 mg eines Gallensäuremethylesters, wobei

$$A\text{-}X = H_2C{=}CH\text{-}\overset{\overset{O}{\|}}{C}\text{-}NH\text{-}(CH_2)_6\text{-}O\text{-}$$

bedeutet, in 4 ml Tetrahydrofuran, gelöst und mit 120 mg 75 %igem Dibenzoylperoxid, gelöst in 0,4 ml Toluol, versetzt. Die Reaktionsmischung wird unter Rühren auf 80°C erhitzt. Zu der Reaktionsmischung werden innerhalb von 4 Stunden 17,01 g N-Vinylpyrrolidon kontinuierlich zudosiert, wobei nach 2 und nach 4 Stunden Reaktionszeit jeweils 120 mg 75 %iges Dibenzoylperoxid, gelöst in 0,4 ml Toluol, zugesetzt werden. Nach erfolgter Zudosierung des N-Vinylpyrrolidons läßt man für zwei Stunden bei 80°C nachreagieren. Anschließend verdünnt man die Reaktionsmischung mit 15 ml THF und versetzt mit 0,5 ml 20 %iger wäßriger Natronlauge. Nach ca. 10 Minuten wird die auftretende Trübung der Reaktionsmischung durch Zusatz von Wasser in Lösung gebracht, dieser Vorgang wird so oft wiederholt, bis keine Trübung der Mischung mehr auftritt. Anschließend wird die Reaktionsmischung mit 50 ml Wasser verdünnt, 24 Stunden gegen entsalztes Wasser dialysiert (cut off: 3500 g/mol) und gefriergetrocknet.

Molares Verhältnis von Gallensäureeinheiten zu N-Vinylpyrrolidon-Einheiten

2 : 3 (ermittelt durch NMR),
gewichtsmittleres Molekulargewicht der unverseiften Substanz:
$M_w$ = 16 000 g/mol (ermittelt mit GPC).

Beispiel 5

In einem Reaktionsgefäß werden unter Stickstoff 1110 mg eines Gallensäuremethylesters, wobei

$$A\text{-}X = H_2C{=}CH\text{-}\overset{\overset{O}{\|}}{C}\text{-}NH\text{-}(CH_2)_6\text{-}O\text{-}$$

bedeutet, in 8 ml Tetrahydrofuran gelöst und mit 150 mg 75 %igem Dibenzoylperoxid, gelöst in 0,75 ml Toluol, versetzt. Die Reaktionsmischung wird unter Rühren für 18 Stunden auf 75°C erhitzt. Anschließend verdünnt man die Reaktionsmischung mit 10 ml THF und versetzt mit 0,5 ml 20 %iger wäßriger Natronlauge. Nach ca. 10 Minuten wird die auftretende Trübung der Reaktionsmischung durch Zusatz von Wasser in Lösung gebracht. Dieser Vorgang wird so oft wiederholt, bis keine Trübung der Mischung mehr auftritt. Anschließend wird die Reaktionsmischung mit 30 ml Wasser verdünnt, 24 Stunden gegen entsalztes Wasser dialysiert (cut off: 3500 g/mol) und gefriergetrocknet.

Gewichtsmittleres Molekulargewicht der unverseiften Substanz:
$M_w$ = 10 000 g/mol (ermittelt mit GPC).

Beispiel 6

In einem Reaktionsgefäß werden unter Stickstoff 386,8 mg eines Gallensäuremethylesters, wobei

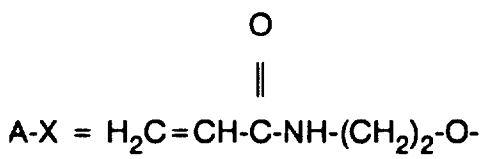

bedeutet, in 2,78 ml Tetrahydrofuran gelöst und mit 52 mg 75 %igem Dibenzoylperoxid, gelöst in 0,5 ml Toluol, versetzt. Die Reaktionsmischung wird unter Rühren für 18 Stunden auf 75°C erhitzt. Anschließend verdünnt man die Reaktionsmischung mit 10 ml THF und versetzt mit 0,5 ml 20 %iger wäßriger Natronlauge. Nach ca. 10 Minuten wird die auftretende Trübung der Reaktionsmischung durch Zusatz von Wasser in Lösung gebracht. Dieser Vorgang wird so oft wiederholt, bis keine Trübung der Mischung mehr auftritt. Anschließend wird die Reaktionsmischung mit 30 ml Wasser verdünnt, 24 Stunden gegen entsalztes Wasser dialysiert (cut off: 3500 g/mol) und gefriergetrocknet.

Gewichtsmittleres Molekulargewicht der unverseiften Substanz:
$M_w$ = 11 000 g/mol (ermittelt mit GPC)

Beispiel 7

In einem Reaktionsgefäß werden unter Stickstoff 340 mg eines Gallensäuremethylesters, wobei

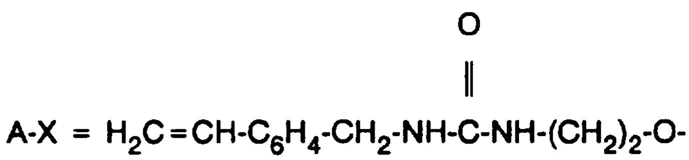

bedeutet, in 2,4 ml Tetrahydrofuran gelöst und mit 45 mg 75 %igem Dibenzoylperoxid, gelöst in 0,5 ml Toluol, versetzt. Die Reaktionsmischung wird unter Rühren für 18 Stunden auf 75°C erhitzt. Anschließend verdünnt man die Reaktionsmischung mit 10 ml THF und versetzt mit 0,5 ml 20 %iger wäßriger Natronlauge. Nach ca. 10 Minuten wird die auftretende Trübung der Reaktionsmischung durch Zusatz von Wasser in Lösung gebracht. Dieser Vorgang wird so oft wiederholt, bis keine Trübung der Mischung mehr auftritt. Anschließend wird die Reaktionsmischung mit 30 ml Wasser verdünnt, 24 Stunden gegen entsalztes Wasser dialysiert (cut off: 3500 g/mol) und gefriergetrocknet.

Beispiel 8

In einem Reaktionsgefäß werden unter Stickstoff 558,8 mg eines Gallensäuremethylesters, wobei

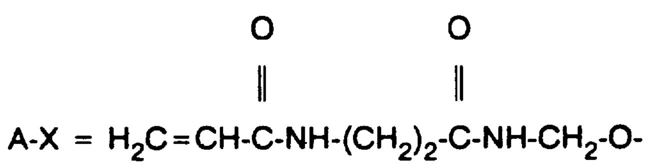

bedeutet, in 4 ml Tetrahydrofuran gelöst und mit 74,5 mg 75 %igem Dibenzoylperoxid, gelöst in 0,8 ml Toluol, versetzt. Die Reaktionsmischung wird unter Rühren für 18 Stunden auf 75°C erhitzt. Anschließend verdünnt man die Reaktionsmischung mit 10 ml THF und versetzt mit 0,5 ml 20 %iger wäßriger Natronlauge. Nach ca. 10 Minuten wird die auftretende Trübung der Reaktionsmischung durch Zusatz von Wasser in Lösung gebracht. Dieser Vorgang wird so oft wiederholt, bis keine Trübung der Mischung mehr auftritt. Anschließend wird die Reaktionsmischung mit 30 ml Wasser verdünnt, 24 Stunden gegen entsalztes Wasser dialysiert (cut off: 3500 g/mol) und gefriergetrocknet.

Gewichtsmittleres Molekulargewicht der unverseiften Substanz:
$M_w$ = 14 000 g/mol (ermittelt mit GPC).

Beispiel 9

In einem Reaktionsgefäß werden unter Stickstoff 437,4 mg eines Gallensäuremethylesters, wobei

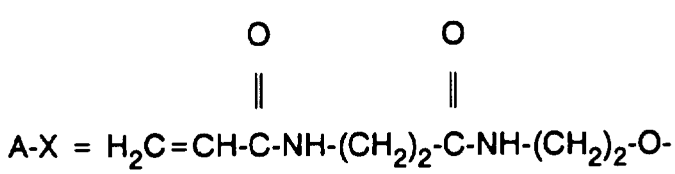

bedeutet, in 3,2 ml Tetrahydrofuran gelöst und mit 62,7 mg 75 %igem Dibenzoylperoxid, gelöst in 0,7 ml Toluol, versetzt. Die Reaktionsmischung wird unter Rühren für 18 Stunden auf 75°C erhitzt. Anschließend verdünnt man die Reaktionsmischung mit 10 ml THF und versetzt mit 0,5 ml 20 %iger wäßriger Natronlauge. Nach ca. 10 Minuten wird die auftretende Trübung der Reaktionsmischung durch Zusatz von Wasser in Lösung gebracht. Dieser Vorgang wird so oft wiederholt, bis keine Trübung der Mischung mehr auftritt. Anschließend wird die Reaktionsmischung mit 30 ml Wasser verdünnt, 24 Stunden gegen entsalztes Wasser dialysiert (cut off: 3500 g/mol) und gefriergetrocknet.

Gewichtsmittleres Molekulargewicht der unverseiften Substanz:
$M_w$ = 13 000 g/mol (ermittelt mit GPC).

Beispiel 10

Das Beispiel wird entsprechend Beispiel 9 durchgeführt, jedoch werden folgende Verbindungen eingesetzt:

424 mg Gallensäuremethylester in 3 ml THF, wobei A-X =

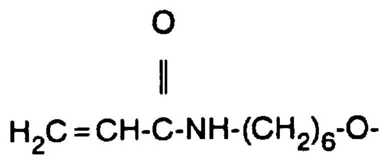

bedeutet
0,424 mg Dodecylmercaptan in 50 µl THF als Regler und
56,52 mg Dibenzoylperoxid (BSO), 75 %ig, in 563,7 µl Toluol.

Das erhaltene Produkt weist ein gewichtsmittleres Molekulargewicht der unverseiften Substanz von $M_w$ = 6900 g/mol (ermittelt mit GPC) auf.

Beispiel 11

Das Beispiel wird entsprechend Beispiel 9 durchgeführt, jedoch werden folgende Verbindungen eingesetzt:

400 mg Gallensäuremethylester in 2,9 ml THF, wobei A-X =

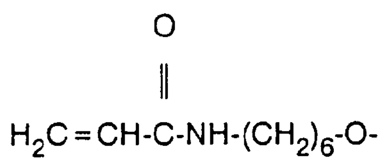

bedeutet und
5,39 mg BSO, 75 %ig, in 53,8 µl Toluol

Das erhaltene Produkt weist ein gewichtsmittleres Molekulargewicht der unverseiften Substanz von $M_w$ = 8800 g/mol (ermittelt mit GPC) auf.

Beispiel 12

Das Beispiel wird entsprechend Beispiel 9 durchgeführt, jedoch werden folgende Verbindungen eingesetzt:

407,3 mg Gallensäuremethylester in 2,95 ml THF, wobei A-X =

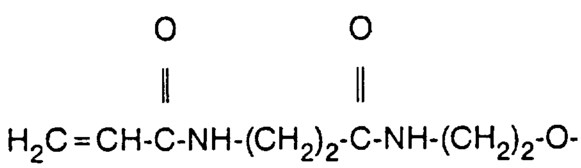

bedeutet,
1,53 mg Glyoxalbisacrylamid in 74,6 µl Methanol als Vernetzer und
58,4 mg BSO, 75 %ig, in 582,4 µl Toluol.

Das erhaltene Produkt weist ein jeweils mittleres Molekulargewicht der unverseiften Substanz von $M_w$ = 5400 g/mol (ermittelt mit GPC) auf.

Beispiel 13

Das Beispiel wird entsprechend Beispiel 9 durchgeführt, jedoch werden folgende Verbindungen eingesetzt:

409 mg Gallensäuremethylester in 2,95 ml THF, wobei A-X =

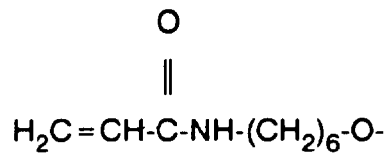

bedeutet,
54,53 mg BSO, 75 %ig, in 543,8 µl Toluol.

Das erhaltene Produkt weist ein gewichtsmittleres Molekulargewicht der unverseiften Substanz von $M_w$ = 7500 g/mol (ermittelt mit GPC) auf.

Beispiel 14

Das Beispiel wird entsprechend Beispiel 9 durchgeführt, jedoch werden folgende Verbindungen eingesetzt:

400,7 mg Gallensäuremethylester in 2,9 ml THF, wobei A-X =

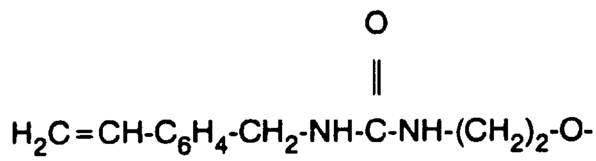

bedeutet,
1,5 mg Glyoxalbisacrylamid als Vernetzer in 70 µl Methanol und
53,4 mg BSO, 75 %ig, in 529 µl Toluol.

Beispiel 15

Das Beispiel wird entsprechend Beispiel 9 durchgeführt, jedoch werden folgende Verbindungen eingesetzt:

416,3 mg Gallensäuremethylester in 2,9 ml THF, wobei A-X =

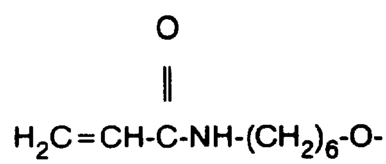

bedeutet, und
0,81 mg tert. Butylperoxydiethylacetat als Initiator in 15,5 µl THF,
Reaktionszeit 21 h.

Das erhaltene Produkt weist ein gewichtsmittleres Molekulargewicht der unverseiften Substanz von $M_w$ = 10700 g/mol (ermittelt mit GPC) auf.

Bei In-vitro und In-vivo Untersuchungen der erfindungsgemäßen Verbindungen vom Typ der polymeren Gallensäuren, die eine hohe Affinität für das Gallensäuretransportsystem besitzen, wurde überraschender Weise gefunden, daß diese die Gallensäureresorption in konzentrationsabhängiger Weise hemmen. Weiterhin konnte festgestellt werden, daß die erfindungsgemäßen Verbindungen selbst nicht resorbiert werden und somit nicht in den enterohepatischen Kreislauf gelangen. Aufgrund dieser Erkenntnis kann nun mit höherer Effizienz in den enterohepatischen Kreislauf eingegriffen werden, als es mit den Resins bislang möglich war.

Bei dem schon als Arzneimittel erhältlichen Resins, z.B. Colestyramin (enthält quartäre Ammoniumgruppen) oder Colestipol (enthält sekundäre bzw. tertiäre Aminogruppen) ist die zweckmäßige Tagesdosis sehr hoch. Sie beträgt z.B. für Colestyramin 12-24 g, Tageshöchstdosis 32 g. Die empfohlene Dosis ist 15-20 g. Weiterhin erschweren Geschmack, Geruch und die hohe Dosierung die Patienten-Compliance. Die bekannten Nebenwirkungen (z. B. Avitaminosen) der Resins gehen auf mangelnde Selektivität zurück. Diese Nebenwirkungen müssen auch bei der Dosierung simultan gegebener Medikamente berücksichtigt werden, aber auch bei einer Gallensäureverarmung, die verschiedene gastrointestinele Störungen (Obstipation, Steatorrhoe) unterschiedlichen Grades hervorrufen.

Für Cholestyramin und Golestipol wurde eine therapeutische Bedeutung durch Kombination mit anderen hypolipidämisch wirkenden Pharmaka wie Fibrate, HMG-CoA-Reduktase-Inhibitoren, Probucol (vgl. z.B. M.N. Cayen, Pharmac. Ther. 29, 187 (1985) und 8th International Symposium on Atherosclerosis, Rome, Oct. 9-13, 1988, Abstracts S. 544, 608, 710) beschrieben, wobei die erzielten Effekte auch die Therapie von schweren Hyperlipidämien ermöglichen. Jedoch sind folgende Merkmale der genannten Präparate und insbesondere von z.B. Colestipol als verbesserungswürdig anzusehen:

1. Die hohen Tagesdosen, die zurückzuführen sind auf eine relativ geringe Bindungsrate bei neutralem pH in isotonen Medium und der (teilweisen) Wiederfreisetzung der adsorbierten Gallensäuren.

2. Die qualitative Verschiebung der Gallensäurezusammensetzung der Galle mit abnehmender Tendenz für Chenodesoxycholsäure und die damit verbundene zunehmende Gefahr für Cholelithiasis.

3. Das Fehlen einer dämpfenden Wirkung auf den Cholesterinstoffwechsel der Darmbakterien.

4. Die zu hohe Bindungsrate von Vitaminen und Pharmaka macht einen Substitutionsbedarf an diesen Stoffen und Blutspiegelkontrollen eventuell notwendig.

5. Die Darreichungsform ist bislang als unzureichend anzusehen.

Durch Inhibition der Gallensäurerückresorption mit Hilfe der erfindungsgemäßen polymeren Gallensäuren im Dünndarm wird auf wesentlich effektivere Weise die im enterohepatischen Kreislauf befindliche Gallensäurekonzentration vermindert, so daß eine Senkung des Cholesterinspiegels im Serum erfolgt. Avitaminosen werden bei Anwendung der erfindungsgemäßen Verbindungen danach ebensowenig beobachtet, wie die Beeinflussung der Resorption anderer Arzneimittel. Auch wird keine negative Wirkung auf die Darmflora gesehen, da die Bindung der erfindungsgemäßen Polymeren an die Darmschleimhaut außerordentlich stabil ist und nach erfolgter Bindung sehr lang anhält. Es ist zudem zu erwarten, daß die bekannten Nebenwirkungen (Obstipation, Steratorrhoe) nicht beobachtet werden.

Schließlich führt der Einsatz hoher Dosierungen nicht zu einer Zellschädigung. Daher kann durch Einsatz der erfindungsgemäßen Verbindungen die sonst übliche Dosierung der Resins erheblich gesenkt werden. Die empfohlene Dosis beträgt zweckmäßigerweise bis zu 10 g pro Tag, vorzugsweise 0,1 bis 5,0 g /Tag, insbesondere 0,3 bis 5,0 g/Tag.

Folgende Methoden wurden angewendet:

HPLC mit FLUORESZENZDETEKTION

| | |
|---|---|
| Geräte: | HPLC-Anlage der Fa. Kontron, bestehend aus drei Pumpen und Mischkammer, Autosampler, UV-Detektor und Auswerteeinheit mit Software MT2.<br>Fluroeszenzdetektor von Merck-Hitachi Da die Proben licht- und temperaturempfindlich sind, wird der Autosampler auf ca. 5°C gekühlt. |
| Mobile Phase: | Laufmittel A: ®Millipore-Wasser (eigene Anlage)<br>Laufmittel B: Acetonitril/Methanol 60:30 |
| Säule: | ®LiChrospher 100 RP-18, 25 mm, 5 µm von Merck |
| Vorsäule: | LiChrospher 60 RP-select B, 4 mm, 5 µm von Merck |
| Flußrate: | 1.3 ml/min |
| Detektion: Excitation: | 340 nm |
| Emission: | 410 nm |

Gradient:

| | |
|---|---|
| 0,00 min | 66 % B |
| 7,50 min | 66 % B |
| 8,00 min | 76 % B |
| 12,50 min | 76 % B |
| 13,00 min | 83 % B |
| 25,00 min | 83 % B |
| 25,50 min | 91 % B |
| 40,00 min | 91 % B |

Enzymatische Bestimmung der Gesamtgallensäure

- In Eppendorfgefäße werden je 900 µl des folgenden Gemisches gegeben:
  6 ml Tetra-Natrium-Diphosphat-Puffer 0,1 M, pH 8,9,
  2 ml NAD-Lösung (4 mg/ml Wasser),
  20 ml Millipore-Wasser
- Dazu werden 30 µl der Probe und 30 µl Enzymlösung pipettiert.
- Enzymlösung: 3-alpha-Hydroxysteroiddehydrogenase 0,5 units/ml
- Die Ansätze werden gemischt und 2 h bei Raumtemperatur inkubiert.
- Anschließend Umfüllen in 1 ml-Einmalküvetten und Messung im Photometer bei 340 nm.
- Für Gallenproben nur bedingt geeignet, da die grüne Farbe stört.

HPLC mit UV-DETEKTION

Geräte: HPLC-Anlage der Fa. Kontron, bestehend aus drei Pumpen und Mischkammer, Autosampler, UV-Detektor und Auswerteeinheit mit Software MT2.

Mobile Phase: Laufmittel A: Ammoniumcarbamatpuffer 0,019 M, mit Phosphorsäure auf pH 4,0 eingestellt.
Laufmittel B: Acetonitril

Säule: LiChrospher 100 RP-8, 25 mm, 5 µm von Merck

Vorsäule: LiChrospher 60 RP-select B, 4 mm, 5 µm von Merck

Flußrate:

| | | |
|---|---|---|
| Gradient: | 0,00 min | 0,8 ml/min |
| | 20,00 min | 0,8 ml/min |
| | 23,00 min | 1,3 ml/min |
| | 51,00 min | 1,3 ml/min |

Detektion: 200 nm (für Präparate zusätzlich bei 254 nm)

Gradient:

| | |
|---|---|
| 0,00 min | 32 % B |
| 8,00 min | 35 % B |
| 17,00 min | 38 % B |
| 20,00 min | 40 % B |
| 24,00 min | 40 % B |
| 30,00 min | 50 % B |
| 45,00 min | 60 % B |

Die in vivo Untersuchung erfolgte wie bei F.G.J. Poelma et al. (J. Pharm. Sci. 78 (4), 285-89, 1989) beschrieben mit einigen Modifikationen.

In den Untersuchungen werden Taurocholat und Taurocholsäure bzw. Cholat und Cholsäure synonym verwendet.

Kanülierung des Gallengangs

Der Gallengang wird freipräpariert und ein Katheter eingebunden (PE 50, Intramedic$^R$). An dessen Ende wurde ein Adapter zur Aufnahme von 100 µl Einmalpipettenspitzen (Brandt) angebracht. In diesen Pipetten wird die Galle gesammelt und nach bestimmten Zeitabständen in ausgewogene Eppendorf-Reaktionsgefäße gefüllt. Nach Versuchsende wird die Galle, wie auch die Mediumproben, ausgewogen und Aliquots im Szintillationszähler vermessen. Dazu werden 10 µl Probe in ein Sarstedt Probengefäß, 58 x 22 mm, pipettiert, mit 10 ml Quickszint 212 (Zinsser GmbH, Frankfurt am Main, Deutschland) versetzt und nach 30 min Abklingzeit in einem Beckman 2800 β-Counter gezählt.

1. Die erfindungsgemäßen Verbindungen, Beispiele 1 bis 15, wurden zusammen mit 10 mM Taurocholat mit $^3$H-Taurocholat oder $^{14}$C-Taurocholat als Tracer in das Darmsegment instilliert und die Perfusionslösung mit Hilfe einer Peristaltikpumpe 2 h umgepumpt. Die Abnahme des Tracers im Darm (Medium) bzw. das Erscheinen des Tracers in der Gallenflüssigkeit (Galle) wurde mit Hilfe von Szintillationsmessungen und HPLC ermittelt. Als Kontrolle wurde 10 mM Taurocholat mit dem Tracer ohne erfindungsgemäße Verbindung instilliert und die Änderung im Darm und in der Gallenflüssigkeit ermittelt (Figuren 1a - 10a und 1b - 10b).

2. In vivo perfundierter Darm

Als Versuchstiere kommen Wistar-Ratten aus eigener Zucht (Tierhaltung Hoechst) mit durchschnittlich 230-290 g Körpergewicht zum Einsatz. Die Versuchstiere werden vor der Narkose (Urethan 1 g/kg i.p.) nicht gehungert. Nach Eintritt der Narkose werden die Tiere auf einem temperierbaren (konstant 37°C) OP-Tisch (Medax) fixiert, auf der Bauchseite geschoren und anschließend wird den Tieren mit einem ca. 7 cm langem Schnitt die Bauchdecke geöffnet. Sodann wird ca. 8 cm von der Ileozökalklappe ein Luer-Adapter (Feinmechanik Hoechst) in den unteren Dünndarm eingebunden, dabei wird der weiterführende Dünndarm abgebunden.

Nun erfolgt eine weitere Ein- und Abbindung des Dünndarms 13-14 cm zum Dünndarmanfang hin. Der Inhalt dieses Darmsegments wird mit 37°C warmer isotonischer Kochsalzlösung vorsichtig ausgespült. In dieses Segment, Endteil Jejunum-Anfang Ileum, wird später die Versuchslösung instilliert.

Zuerst wird der Pumpenschlauch aus den 2 ml Instillationslösung (10 mM Taurocholat, Präparat in gegebener Konzentration, Tracer: 3.5 µCi [$^3$H(G)]-Taurocholsäure, NET-322, Lot 2533-081, DuPont de Nemour GmbH, Dreieich, Deutschland, gelöst in Phosphat-gepufferter isotonischer Kochsalzlösung gefüllt (Silikon Tubing A, 0,5 mm Desaga, Heidelberg, Deutschland, Best. Nr. 132020). Danach wird der Pumpenschlauch mit zwei Luer-Adaptern an das Darmsegment angeschlossen und die Restlösung über einen Dreiweghahn (Pharmascal K 75a) und einer 2 ml-Einwegspritze (Chirana) eingefüllt. Unmittelbar danach wird die Peristaltikpumpe (LKB Multiperpex 2115) eingeschaltet, und das Medium mit 0,25 ml/min umgepumpt. In regelmäßigen Abständen wird aus einem in den Kreislauf integrierten Infusionsschlauch mittels einer Hamilton-Spritze und einer Kanüle (Termo 0,4 x 20) eine Probe für die Messung der Aktivität (Abnahme Radioaktivität im Darm = Resorptionsrate) entnommen.

Zum Nachweis der prolongierten Wirkung der oligomeren bzw. polymeren Gallensäuren (Figuren 11a + b) wird in diesem speziellen Versuchsdesign der Abstrom (Darm) und das Anfüllen (Galle) des radioaktiven Tracers während der ersten (Inst. I) mit Inhibitor und während der zweiten Instillation (Inst. II) ohne den Inhibitor geprüft.

**Patentansprüche**

**1.** Polymere und oligomere Gallensäuren, herstellbar durch Polymerisation von monomeren Gallensäuren der Formel I

$$G - X - A \qquad (I)$$

in der

| | |
|---|---|
| G | ein Gallensäurerest oder -derivat, |
| X | eine Brückengruppe und |
| A | eine polymersierbare, ethylenisch ungesättigte Gruppe bedeutet, |

oder durch Copolymerisation mit einem, eine polymerisierbare, ethylenisch ungesättigte Doppelbindung aufweisenden Monomeren, insbesondere durch Copolymerisation mit einem Monomeren der Formel IV

$$\begin{array}{c} R^9 \\ | \\ H_2C = C - R^{10}, \end{array} \qquad IV$$

worin

$R^9$ Wasserstoff oder Methyl und

$R^{10}$

$$\overset{\displaystyle O}{\overset{\|}{-C}}-O-R^{11},\ \overset{\displaystyle O}{\overset{\|}{-C}}-NR^{12}R^{13},\ -O-\overset{\displaystyle O}{\overset{\|}{C}}-R^{14},\ -CN,\ -O-R^{15},$$

Wasserstoff, Halogen, insbesondere Chlor, Brom oder Iod, $-SO_3H$ oder $-O-(CH_2-CH_2O)_nR^{16}$, worin

| | |
|---|---|
| $R^{11}$ | Wasserstoff, $(C_1-C_{10})$-Alkyl, $(C_1-C_{10})$-mono-Hydroxyalkyl oder $-(CH_2CH_2-O-)_nR^{16}$, |
| $R^{12}$, $R^{13}$, $R^{15}$ und $R^{16}$ | gleich oder verschieden sind und $(C_1-C_{10})$-Alkyl, |
| $R^{14}$ | $(C_1-C_{18})$-Alkyl und |
| n | 1 bis 50 |

bedeuten,

oder durch Copolymerisation mit N-Vinylpyrrolidon oder dessen Derivaten und/oder durch Copolymerisation mit ethylenisch ungesättigten Dicarbonsäureanhydriden und ethylenisch ungesättigten Dicarbonsäuren mit jeweils 2 bis 6 C-Atomen, deren Estern oder Halbestern, wobei als Ester Alkylester mit 1-6 C-Atomen, Cycloalkylester mit 5 bis 8 C-Atomen, Benzyl- oder Phenylester zu verstehen sind.

**2.** Polymere und Oligomere nach Anspruch 1, dadurch gekennzeichnet, daß

G eine freie Gallensäure bzw. ihr Alkalisalz oder eine am Ring D veresterte Gallensäure ist, die über ihren Ring A, B oder C, vorzugsweise über Ring A, verbunden ist mit der Gruppe

X, für die bevorzugt die Formel II gilt

$$(Y)_o - (Z)_p \qquad (II),$$

worin

Y benachbart zu G steht und

$$-O-,\ -NR'-,\ -O-\overset{\displaystyle O}{\overset{\|}{C}}-,\ -NR'-\overset{\displaystyle O}{\overset{\|}{C}}-,$$

Z $(C_1-C_{12})$-Alkylen oder $(C_7-C_{13})$-Aralkylen, wobei einzelne, bevorzugt 1 bis 4, Methylengruppen in der Alkylenkette des Alkylen- oder Aralkylenrestes durch Gruppen wie

$$-O-,\ -NR'-,\ -NR'-\underset{\displaystyle O}{\underset{\|}{C}}-,\ -O-\underset{\displaystyle O}{\underset{\|}{C}}-\ \text{oder}$$

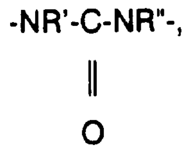

bevorzugt eine Gruppe eines Types, ersetzt sein können,

o und p unabhängig voneinander null oder 1, wobei o und p nicht gleichzeitig null sind

A eine ethylenisch ungesättigte Gruppe der Formel

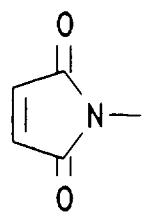

oder

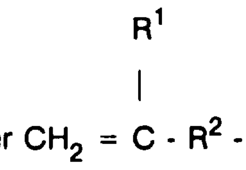

bedeuten, worin

$R^1$ Wasserstoff oder $CH_3$ und

$R^2$

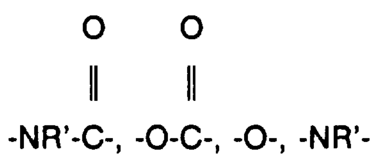

oder eine Einfachbindung bedeutet, wobei die Carbonylgruppen benachbart zur C-C-Doppelbindung stehen,

R' und R" unabhängig voneinander Wasserstoff oder ($C_1$-$C_6$)-Alkyl, bevorzugt ($C_1$-$C_3$)-Alkyl bedeuten.

**3.** Polymere und Oligomere nach den Ansprüchen 1 oder 2, dadurch gekennzeichnet, daß

G der Formel III entspricht

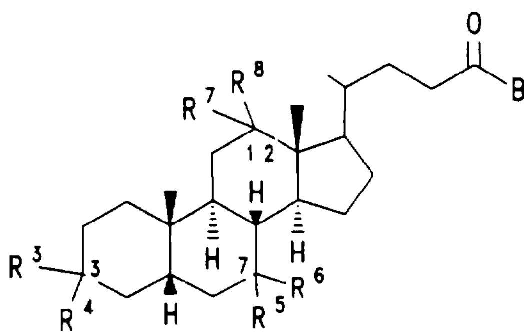

(III)

worin

$R^3$ bis $R^8$ unabhängig voneinander Wasserstoff, OH, $NH_2$ oder eine mit einer OH-Schutzgruppe geschützte OH-Gruppe und einer der Reste $R^3$ bis $R^6$ eine Bindung zur Gruppe X bedeuten, wobei diese Bindung von den Positionen 3 ($R^3$ oder $R^4$) oder 7 ($R^5$ oder $R^6$) bevorzugt ist die β-Position, ausgeht und die jeweils andere Position 7 oder 3 eine OH-Gruppe oder eine geschützte OH-Gruppe trägt,

B -OH, -O-Alkali, -O-Erdalkali, -O-($C_1$-$C_{12}$)-Alkyl, -O-Allyl oder -O-Benzyl bedeutet, bevorzugt -OH, -O-Alkali, -O-($C_1$-$C_6$)-Alkyl, -O-Allyl oder -O-Benzyl, wobei Alkyl sowohl n-Alkyl wie iso-Alkyl bedeutet und wobei die gebildete Estergruppe

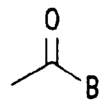

sowohl sauer wie auch basisch verseifbare Ester darstellt,

Y

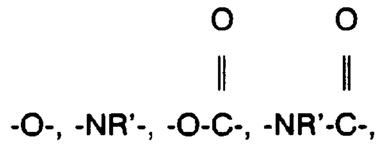

Z $(C_1-C_{12})$-Alkylen, $(C_7-C_{13})$-Aralkylen, wobei 1 bis 3 Methylengruppen in der Alkylenkette durch die Gruppen

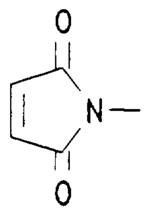

oder 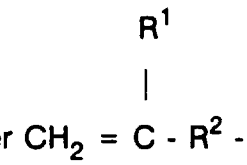

ersetzt sind und

o und p unabhängig vonein ull sind,

A

bedeuten, wobei

$R^1$ Wasserstoff oder $CH_3$ und

$R^2$

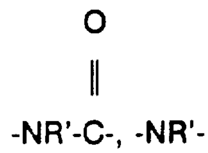

oder eine Einfachbindung bedeutet, worin

R' und R'' unabhängig voneinander Wasserstoff oder $(C_1-C_6)$-Alkyl bedeuten.

**4.** Polymere und Oligomere nach einem der Ansprüche 1 bis 3, dadurch gekennzeichnet, daß als Monomere Verbindungen gemäß der Formel IV (Meth)acrylsäure, (Meth)acrylsäureester, Acrylamid und dessen Derivate, Carbonsäurevinylester mit 3-20 C-Atomen oder N-Vinylpyrrolidon und dessen Derivate gelten.

**5.** Polymere und Oligomere nach einem der Ansprüche 1 bis 4, dadurch gekennzeichnet, daß das gewichtsmittlere Molekulargewicht bei bis zu 250 000 g/mol, besonders bevorzugt zwischen 2000 und 100 000 g/mol, insbesondere bevorzugt zwischen 3000 und 60 000 g/mol liegt.

**6.** Polymere und Oligomere nach einem der Ansprüche 1 bis 5, dadurch gekennzeichnet, daß im Falle von Copolymeren das molare Verhältnis von Gallensäureeinheiten zu copolymerisierten Monomereinheiten zwischen 300:1 und 1:300, besonders bevorzugt zwischen 150:1 und 1:150 liegt.

**7.** Polymere und Oligomere nach einem der Ansprüche 1 bis 6, dadurch gekennzeichnet, daß die Vernetzung durch eine Copolymerisation mit mehrfach ethylenisch ungesättigten Monomeren erfolgt.

**8.** Polymere und Oligomere nach Anspruch 7, dadurch gekennzeichnet, daß die Vernetzung mit mehrfach ethylenisch ungesättigten Acryl- und Methacrylsäurederivaten durchgeführt wird.

**9.** Polymere und Oligomere nach einem der Ansprüche 7 oder 8, dadurch gekennzeichnet, daß die Vernetzung mit Säureamiden der allgemeinen Formel V

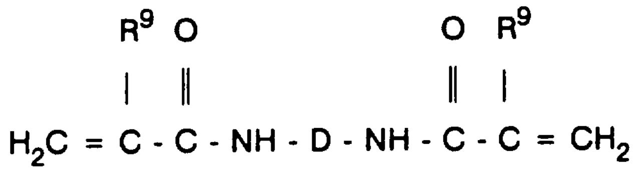 V

worin

R⁹ Wasserstoff oder Methyl und
D $-(CHE)_m-$ bedeutet,
wobei
m 1 bis 10 und
E Wasserstoff oder OH bedeutet,
durchgeführt wird.

**10.** Verfahren zur Herstellung der polymeren und oligomeren Gallensäuren nach den Ansprüchen 1 bis 9, dadurch gekennzeichnet, daß man Verbindungen der Formel I oder mindestens eine Verbindung der Formel I mit einer Verbindung der Formel IV und/oder N-Vinyl-pyrrolidon oder dessen Derivaten und/oder einer ethylenisch ungesättigten Dicarbonsäure mit 2 bis 6 C-Atomen und/oder einem ethylenisch ungesättigten Dicarbonsäureanhydrid mit 2 bis 6 C-Atomen, und/oder einem ethylenisch ungesättigten Dicarbonsäurehalbester mit 2 bis 6 C-Atomen unter Verwendung von mindestens einem Radikalbildner bei Temperaturen unter 250°C polymerisiert.

**11.** Verfahren nach Anspruch 10, dadurch gekennzeichnet, daß die Polymerisation in Suspension, in Substanz oder in Lösung durchgeführt wird.

**12.** Verfahren nach Anspruch 10, dadurch gekennzeichnet, daß die Polymerisation in einem Lösungsmittel durchgeführt wird, in dem das oder die Monomere löslich sind.

**13.** Verfahren nach Anspruch 10, dadurch gekennzeichnet, daß die Temperatur der Polymerisation im Bereich von 40 bis 100°C liegt.

**14.** Polymere und oligomere Gallensäuren gemäß den Ansprüchen 1-9 zur Anwendung als Arzneimittel zur Beeinflussung des enterohepatischen Kreislaufes, der Gallensäuren, der Lipidresorption und des Serumcholesterinspiegels.

**15.** Polymere und oligomere Gallensäuren gemäß den Ansprüchen 1 bis 9 zur zur Anwendung als Arzneimittel zur konzentrationsabhängigen Hemmung der Gallensäureresorption im gastrointestinalen Trakt.

**16.** Polymere und oligomere Gallensäuren gemäß den Ansprüchen 1 bis 6 zur zur Anwendung als Arzneimittel zur nicht systemischen Senkung erhöhter Serumcholesterin- bzw. Blutfettwerte zur Prävention arteriosklerotischer Erscheinungen.

**17.** Kombination von polymeren und oligomeren Gallensäuren gemäß den Ansprüchen 1 - 6 mit systemisch wirkenden, den Lipidmetabolismus beeinflußenden Wirkstoffen zur Anwendung als Arzneimittel.

**Claims**

**1.** A polymeric or oligomeric bile acid, which can be prepared by polymerization of a monomeric bile acid of the formula I

G-X-A (I)

in which

G is a bile acid radical or derivative,
X is a bridge group and
A is a polymerizable, ethylenically unsaturated group,
or by copolymerization with a monomer containing a polymerizable, ethylenically unsaturated double bond, in particular by copolymerization with a monomer of the formula

IV

$$H_2C = C(R^9) - R^{10}, \qquad IV$$

in which

| | |
|---|---|
| $R^9$ | is hydrogen or methyl and |
| $R^{10}$ | is |

$$-C(=O)-O-R^{11},\quad -C(=O)-NR^{12}R^{13},\quad -O-C(=O)-R^{14},\quad -CN,\quad -O-R^{15},$$

hydrogen, halogen, in particular chlorine, bromine or iodine, $-SO_3H$ or $-O-(CH_2-CH_2O)_nR^{16}$, in which

| | |
|---|---|
| $R^{11}$ | is hydrogen, $(C_1-C_{10})$-alkyl, $(C_1-C_{10})$-mono-hydroxyalkyl or $-(CH_2CH_2-O-)_nR^{16}$, |
| $R^{12}$, $R^{13}$, $R^{15}$ and $R^{16}$ | are identical or different and are $(C_1-C_{10})$-alkyl, |
| $R^{14}$ | is $(C_1-C_{18})$-alkyl and |
| n | is 1 to 50, |

or by copolymerization with N-vinylpyrrolidone or its derivatives,

and/or by copolymerization with ethylenically unsaturated dicarboxylic anhydrides and ethylenically unsaturated dicarboxylic acids each having 2 to 6 carbon atoms, their esters or half esters, esters being understood as alkyl esters having 1-6 carbon atoms, cycloalkyl esters having 5 to 8 carbon atoms, benzyl esters or phenyl esters.

**2.** A polymer or oligomer as claimed in claim 1, wherein

| | |
|---|---|
| G | is a free bile acid or its alkali metal salt or a bile acid esterified on ring D and which is bonded via its ring A, B or C, preferably via ring A, to the group |
| X, | to which the formula II preferably applies |

$$(Y)_o-(Z)_p \qquad (II),$$

in which

| | |
|---|---|
| Y | is adjacent to G and is |

$$-O-,\quad -NR'-,\quad -O-C(=O)-,\quad -NR'-C(=O)-,$$

| | |
|---|---|
| Z | is $(C_1-C_{12})$ -alkylene or $(C_7-C_{13})$-aralkylene, where individual methylene groups, preferably 1 to 4, in the alkylene chain of the alkylene or aralkylene radical can be replaced by groups such as |

$$-O-,\quad -NR'-,\quad -NR'-C(=O)-,\quad -O-C(=O)-$$

or

$$-NR'-C(=O)-NR''-,$$

preferably a group of one type,

| | |
|---|---|
| o and p | independently of one another are zero or 1, where o and p are not simultaneously zero, |
| A | is an ethylenically unsaturated group of the formula |

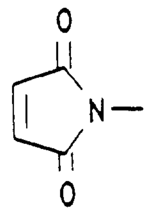

or

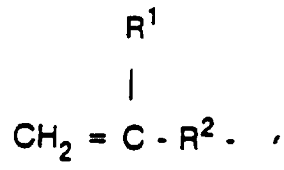

in which

R¹ is hydrogen or $CH_3$ and

R² is

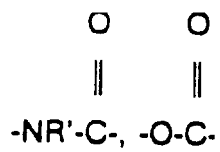

, -O-, -NR'-

or a single bond, where the carbonyl groups are adjacent to the C-C double bond,

R' and R" independently of one another are hydrogen or ($C_1$-$C_6$)-alkyl, preferably ($C_1$-$C_3$)-alkyl.

**3.** A polymer or oligomer as claimed in claim 1 or 2, wherein

G corresponds to the formula III

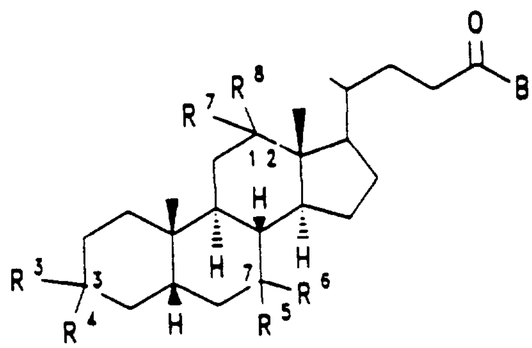

(III)

in which

$R^3$ to $R^8$ independently of one another are hydrogen, OH, $NH_2$ or an OH group protected by an OH protective group and one of the radicals $R^3$ to $R^6$ is a bond to the group X, where this bond starts from the positions 3 ($R^3$ or $R^4$) or 7 ($R^5$ or $R^6$), preferably the β-position, and the other position 7 or 3 in each case carries an OH group or a protected OH group,

B is -OH, -O-alkali metal, -O-alkaline earth metal, -O- ($C_1$-$C_{12}$) -alkyl, -O-allyl or -O-benzyl, preferably -OH, -O-alkali metal, -O-($C_1$-$C_6$)-alkyl, -O-allyl or -O-benzyl, where alkyl is either n-alkyl or iso-alkyl and where the ester group formed

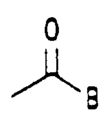

is esters which can be hydrolyzed both by acid and by base,

Y is

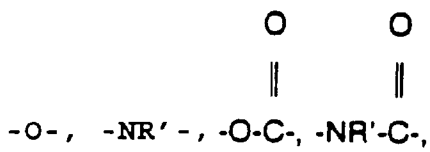

Z is ($C_1$-$C_{12}$)-alkylene, ($C_7$-$C_{13}$) -aralkylene, where 1 to 3 methylene groups in the alkylene chain are replaced by the groups

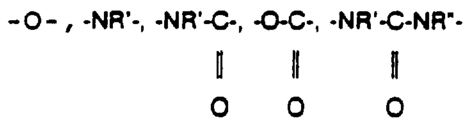

and

o and p independently of one another are zero or 1, where o and p are not simultaneously zero,

A is

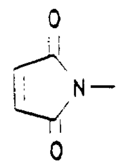

or

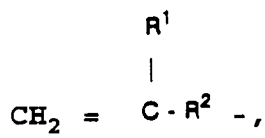

where

$R^1$ is hydrogen or $CH_3$ and

$R^2$ is

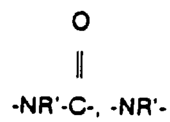

or a single bond, in which

R' and R" independently of one another are hydrogen or ($C_1$-$C_6$) -alkyl.

**4.** A polymer or oligomer as claimed in any one of claims 1 to 3, wherein the monomers are compounds according to the formula IV (meth)acrylic acid, (meth)acrylic acid esters, acrylamide and its derivatives, carboxylic acid vinyl esters having 3-20 carbon atoms or N-vinylpyrrolidone and its derivatives.

**5.** A polymer or oligomer as claimed in any one of claims 1 to 4, wherein the weight-average molecular weight is up to 250,000 g/mol, particularly preferably between 2,000 and 100,000 g/mol, very particularly preferably between 3,000 and 60,000 g/mol.

**6.** A polymer or oligomer as claimed in any one of claims 1 to 5, wherein in the case of copolymers the molar ratio of bile acid units to copolymerized monomer units is between 300:1 and 1:300, particularly preferably between 150:1 and 1:150.

**7.** A polymer or oligomer as claimed in any one of claims 1 to 6, wherein the crosslinking is carried out by means of copolymerization with ethylenically polyunsaturated monomers.

**8.** A polymer or oligomer as claimed in claim 7, wherein the crosslinking is carried out with ethylenically polyunsaturated acrylic acid and methacrylic acid derivatives.

**9.** A polymer or oligomer as claimed in either of claims 7 or 8, wherein the crosslinking is carried out with acid amides of the formula V

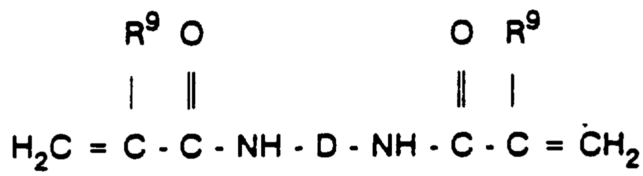

V

in which

R^9 is hydrogen or methyl and
D is - $(CHE)_m$-,
where
m is 1 to 10 and
E is hydrogen or OH.

**10.** A process for the preparation of the polymeric and oligomeric bile acids as claimed in claims 1 to 9, which comprises polymerizing compounds of the formula I or at least one compound of the formula I at temperatures below 250°C with a compound of the formula IV and/or N-vinylpyrrolidone or its derivatives and/or an ethylenically unsaturated dicarboxylic acid having 2 to 6 carbon atoms and/or an ethylenically unsaturated dicarboxylic anhydride having 2 to 6 carbon atoms, and/or an ethylenically unsaturated dicarboxylic acid half ester having 2 to 6 carbon atoms using at least one free radical initiator.

**11.** The process as claimed in claim 10, wherein the polymerization is carried out in suspension, in substance or in solution.

**12.** The process as claimed in claim 10, wherein the polymerization is carried out in a solvent in which the monomer or monomers are soluble.

**13.** The process as claimed in claim 10, wherein the temperature of the polymerization is in the range from 40 to 100°C.

**14.** The polymeric and oligomeric bile acids as claimed in claims 1-9 for use as pharmaceuticals for affecting the enterohepatic circulation, bile acids, lipid absorption and the serum cholesterol level.

**15.** The polymeric and oligomeric bile acids as claimed in claims 1 to 9 for use as pharmaceuticals for the concentration-dependent inhibition of bile acid absorption in the gastrointestinal tract.

**16.** The polymeric and oligomeric bile acids as claimed in claims 1 to 6 for use as pharmaceuticals for non-systemic reduction of elevated serum cholesterol or blood lipid values for the prevention of arteriosclerotic phenomena.

**17.** A combination of polymeric and oligomeric bile acids as claimed in claims 1-6 with systemically acting active compounds which affect lipid metabolism for use as a pharmaceutical.

## Revendications

**1.** Acides biliaires polymères et oligomères, qui peuvent être obtenus par polymérisation d'acides biliaires monomères de formule I

$$G\text{-}X\text{-}A \qquad (I)$$

dans laquelle

G représente un reste ou dérivé d'acide biliaire,
X représente un groupe pontant et
A représente un groupe polymérisable, à insaturation éthylénique,
ou par copolymérisation avec un monomère comportant une double liaison polymérisable, à insaturation éthylénique, en particulier par copolymérisation avec un monomère de formule IV

$$H_2C=C(R^9)-R^{10} \qquad (IV)$$

dans laquelle

$R^9$ représente un atome d'hydrogène ou le groupe méthyle et

$R^{10}$ représente

$$-C(=O)-O-R^{11},\quad -C(=O)-NR^{12}R^{13},\quad -O-C(=O)-R^{14},\quad -CN,\quad -O-R^{15};$$

un atome d'hydrogène ou d'halogène, en particulier de chlore, brome ou iode, $-SO_3H$ ou $-O-(CH_2-CH_2O)_nR^{16}$,

$R^{11}$ représentant un atome d'hydrogène ou un groupe alkyle en $C_1$-$C_{10}$, monohydroxyalkyle en $C_1$-$C_{10}$ ou $-(CH_2CH_2\ -O-)_nR^{16}$

$R^{12}$, $R^{13}$, $R^{15}$ et $R^{16}$ étant identiques ou différents et représentant un groupe alkyle en $C_1$-$C_{10}$,

$R^{14}$ représentant un groupe alkyle en $C_1$-$C_{18}$ et

n allant de 1 à 50,

ou par copolymérisation avec la N-vinylpyrrolidone ou ses dérivés,

et/ou par copolymérisation avec des anhydrides dicarboxyliques à insaturation éthylénique et des acides carboxyliques à insaturation éthylénique, ayant chacun de 2 à 6 atomes de carbone; leurs esters ou hémiesters, étant spécifié que par esters il faut entendre des esters alkyliques ayant de 1 à 6 atomes de carbone, des esters cycloalkyliques ayant de 5 à 8 atomes de carbone, les esters benzyliques ou phényliques.

**2.** Polymères et oligomères selon la revendication 1, caractérisés en ce que

G représente un acide biliaire libre ou un de ses sels alcalins, ou un acide biliaire estérifié sur le cycle D, qui est lié par son cycle A ou B, de préférence par son cycle A, au groupe

X, correspondant de préférence à la formule II

$$(Y)_o - (Z)_p \qquad (II)$$

dans laquelle

Y est contigu à G et représente

$$-O-,\quad -NR'-,\quad -O-C(=O)-,\quad -NR'-C(=O)-,$$

Z représente un radical alkylène en $C_1$-$C_{12}$ ou aralkylène en $C_7$-$C_{13}$, des, de préférence 1 à 4, groupes méthylène individuels dans la chaîne alkylène du radical alkylène ou aralkylène pouvant être remplacés par des groupes tels que

$$-O-,\quad -NR'-,\quad -NR'-C(=O)-,\quad -O-C(=O)-\ \text{ou}\ -NR'-C(=O)-NR''-,$$

de préférence un groupe d'un seul type,

o et p représentent, indépendamment l'un de l'autre, zéro ou 1, o et p n'étant pas simultanément zéro,

A représente un groupe à insaturation éthylénique de formule

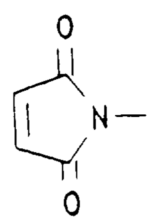

ou un groupe

$$CH_2=\overset{R^1}{\overset{|}{C}}-R^2-$$

dans lequel

$R^1$ représente un atome d'hydrogène ou $CH_3$ et

$R^2$ représente

$$-NR'-\overset{O}{\overset{\|}{C}}-,\quad -O-\overset{O}{\overset{\|}{C}}-,\quad -O-,\quad -NR'-$$

ou une liaison simple, les groupes carbonyle étant voisins de la double liaison C-C,

R' et R" représentent, indépendamment l'un de l'autre, un atome d'hydrogène ou un groupe alkyle en $C_1$-$C_6$, de préférence un groupe alkyle en $C_1$-$C_3$.

**3.** Polymères et oligomères selon la revendication 1 ou 2, caractérisés en ce que G correspond à la formule III

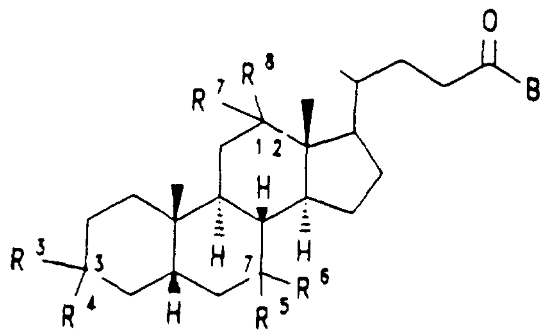

(III)

dans laquelle

$R^3$ à $R^8$ représentent, indépendamment les uns des autres, un atome d'hydrogène ou un groupe OH, $NH_2$, ou un groupe OH protégé par un groupe protecteur de fonction OH, et l'un des radicaux $R^3$ à $R^6$ représente une liaison au groupe X, cette liaison partant des positions 3 ($R^3$ ou $R^4$) ou 7 ($R^5$ ou $R^6$), la position β étant préférée, et l'autre position respective 7 ou 3 portant un groupe OH ou un groupe OH protégé,

B représente -OH, -O-métal alcalin, -O-métal alcalino-terreux, un groupe -O-alkyle en $C_1$-$C_{12}$, -O-allyle ou -O-benzyle, de préférence -OH, -O-métal alcalin, un groupe -O-alkyle en $C_1$-$C_6$, -O-allyle ou -O-benzyle, le groupe alkyle représentant aussi bien un groupe n-alkyle qu'un groupe isoalkyle, et le groupe ester formé

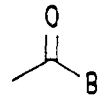

représentant aussi bien un ester saponifiable en présence d'acide qu'un ester saponifiable en présence de base,

Y représente

$$-O-,\quad -NR'-,\quad -O-\overset{O}{\overset{\|}{C}}-,\quad -NR'-\overset{O}{\overset{\|}{C}}-,$$

Z représente un groupe alkylène en $C_1$-$C_{12}$, aralkylène en $C_7$-$C_{13}$, 1 à 3 groupes méthylène dans la chaîne alkylène pouvant être remplacés par les groupes

$$-O-,\quad -NR'-,\quad -NR'-\underset{O}{\underset{\|}{C}}-,\quad -O-\underset{O}{\underset{\|}{C}}-,\quad -NR'-\underset{O}{\underset{\|}{C}}-NR''-,$$

et

o et p représentent, indépendamment l'un de l'autre, zéro ou 1, o et p n'étant pas simultanément zéro,

A représente

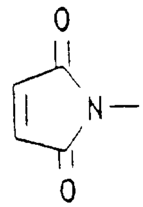

ou un groupe

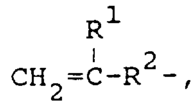

$R^1$ représentant un atome d'hydrogène ou $CH_3$ et

$R^2$ représentant

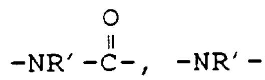

ou une liaison simple,

R' et R" représentant, indépendant l'un de l'autre, un atome d'hydrogène ou un groupe alkyle en $C_1$-$C_6$.

**4.** Polymères et oligomères selon l'une des revendications 1 à 3, caractérisés en ce que, en tant que composés monomères de formule IV, il s'agit de l'acide (méth)acrylique, d'esters d'acides (méth)acrylique, d'acrylamide et de ses dérivés, d'esters vinyliques d'acides carboxyliques ayant de 3 à 20 atomes de carbone, ou de la N-vinylpyrrolidone et de ses dérivés.

**5.** Polymères et oligomères selon l'une des revendications 1 à 4, caractérisés en ce que la masse moléculaire moyenne en poids va jusqu'à 250 000 g/mole, de préférence est comprise entre 2 000 et 100 000 g/mole, et de façon particulièrement préférée se situe entre 3 000 et 60 000 g/mole.

**6.** Polymères et oligomères selon l'une des revendications 1 à 5, caractérisés en ce que, dans le cas des copolymères, le rapport molaire des motifs acide biliaire aux motifs monomères à copolymériser est compris entre 300:1 et 1:300, et se situe de façon particulièrement préférée entre 150:1 et 1:150.

**7.** Polymères et oligomères selon l'une des revendications 1 à 6, caractérisés en ce que la réticulation s'effectue par une copolymérisation avec des monomères à plusieurs insaturations éthyléniques.

**8.** Polymères et oligomères selon la revendication 7, caractérisés en ce que la réticulation est effectuée avec des dérivés d'acides acrylique et méthacrylique à plusieurs insaturations éthyléniques.

**9.** Polymères et oligomères selon la revendication 7 ou 8, caractérisés en ce que la réticulation est effectuée avec des amides de formule générale V

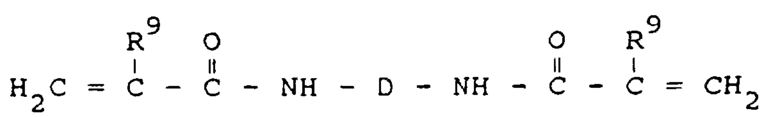 (V)

dans laquelle

$R^9$ représente un atome d'hydrogène ou le groupe méthyle, et

D représente $-(CHE)_m-$,

m allant de 1 à 10, et

E représentant un atome d'hydrogène ou OH.

**10.** Procédé pour la préparation des acides biliaires polymères ou oligomères selon les revendications 1 à 9, caractérisé

en ce que l'on polymérise à des températures inférieures à 250°C des composés de formule I ou au moins un composé de formule I avec un composé de formule IV et/ou la N-vinylpyrrolidone ou ses dérivés et/ou un acide dicarboxylique ayant de 2 à 6 atomes de carbone, à insaturation éthylénique, et/ou un anhydride dicarboxylique ayant de 2 à 6 atomes de carbone, à insaturation éthylénique, et/ou un hémi-ester d'acide dicarboxylique ayant de 2 à 6 atomes de carbone, à insaturation éthylénique, en utilisant au moins un générateur de radicaux.

**11.** Procédé selon la revendication 10, caractérisé en ce que la polymérisation est effectuée en suspension, en masse ou en solution.

**12.** Procédé selon la revendication 10, caractérisé en ce que la polymérisation est effectuée dans un solvant dans lequel le ou les monomères sont solubles.

**13.** Procédé selon la revendication 10, caractérisé en ce que la température de la polymérisation se situe dans la plage allant de 40 à 100°C.

**14.** Acides biliaires polymères ou oligomères selon les revendications 1 à 9, pour utilisation en tant que médicament destiné à exercer un effet sur le cycle entéro-hépatique, les acides biliaires, la résorption des lipides et le taux de cholestérol sérique.

**15.** Acides biliaires polymères ou oligomères selon les revendications 1 à 9, pour utilisation en tant que médicament destiné à l'inhibition, en fonction de la concentration, de la résorption des acides biliaires dans le tractus gastro-intestinal.

**16.** Acides biliaires polymères ou oligomères selon les revendications 1 à 6, pour utilisation en tant que médicament destiné à l'abaissement non systémique de taux augmentés de cholestérol sérique ou de graisse dans la sang, pour la prévention de manifestations artérioscléreuses.

**17.** Association d'acides biliaires polymères ou oligomères selon les revendications 1 à 6 avec des substances actives agissant sur le métabolisme des lipides et à action systémique, pour utilisation en tant que médicament.

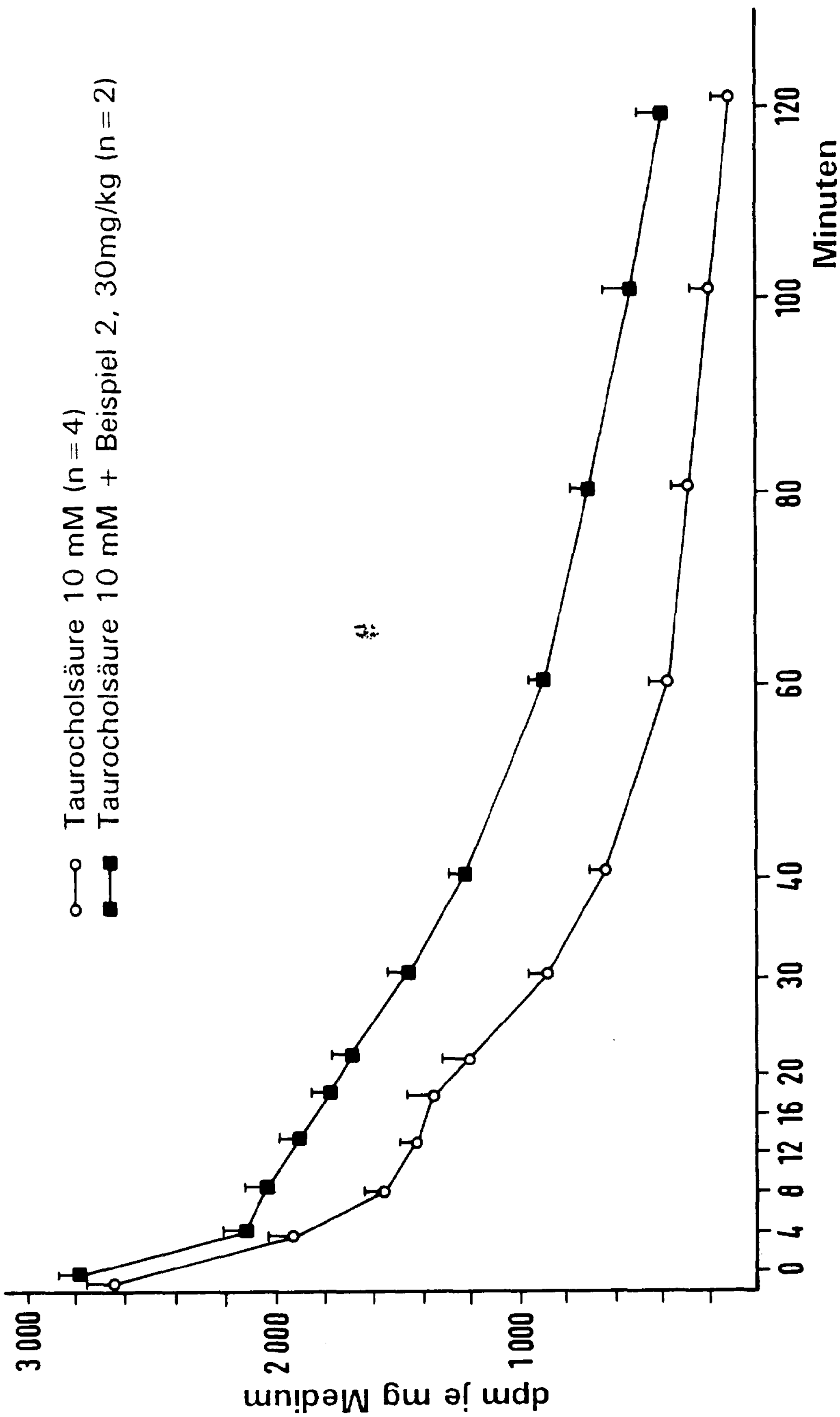
Taurocholsäure 10 mM (n = 4)
Taurocholsäure 10 mM + Beispiel 2, 30mg/kg (n = 2)
dpm je mg Medium
3 000
2 000
1000
0 4 8 12 16 20 30 40 60 80 100 120
Minuten

Fig. 1a

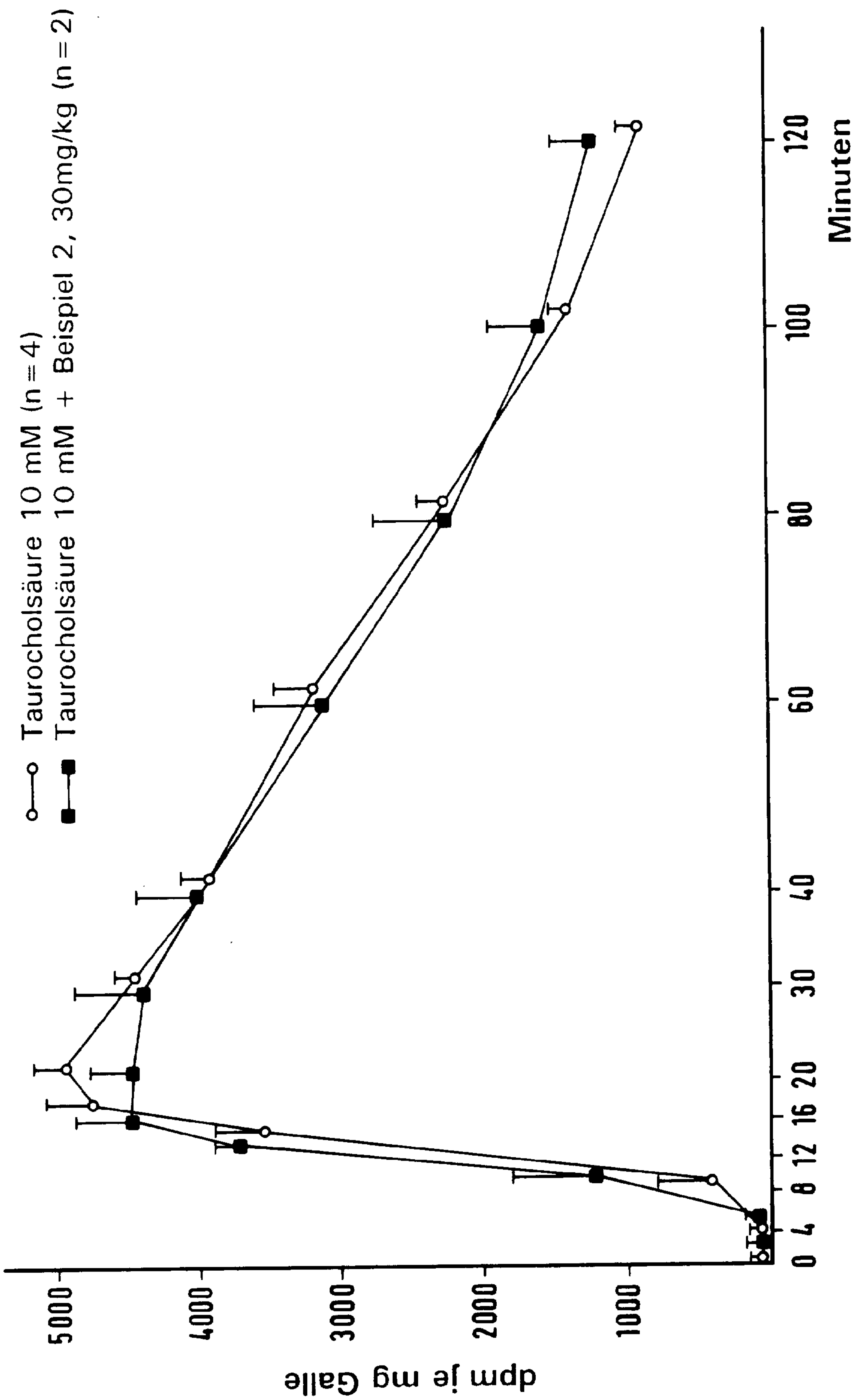
Taurocholsäure 10 mM (n = 4)
Taurocholsäure 10 mM + Beispiel 2, 30mg/kg (n = 2)
dpm je mg Galle
5000
4000
3000
2000
1000
0
4
8
12
16
20
30
40
60
80
100
120
Minuten

Fig. 1b

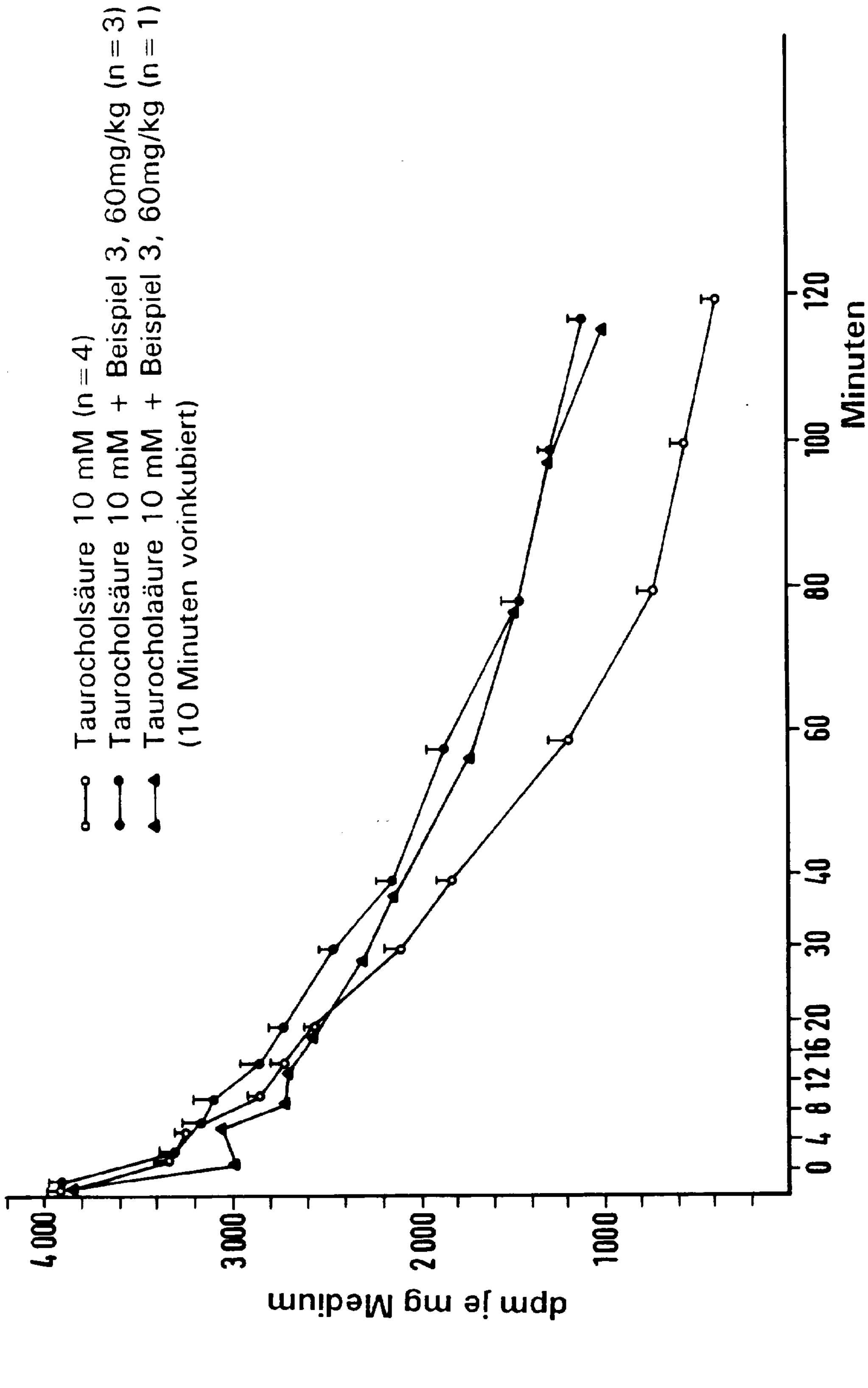
Taurocholsäure 10 mM (n = 4)
Taurocholsäure 10 mM + Beispiel 3, 60mg/kg (n = 3)
Taurocholaäure 10 mM + Beispiel 3, 60mg/kg (n = 1)
(10 Minuten vorinkubiert)
dpm je mg Medium
4 000
3000
2000
1000
0 4 8 12 16 20
30
40
60
80
100
120
Minuten

Fig. 2a

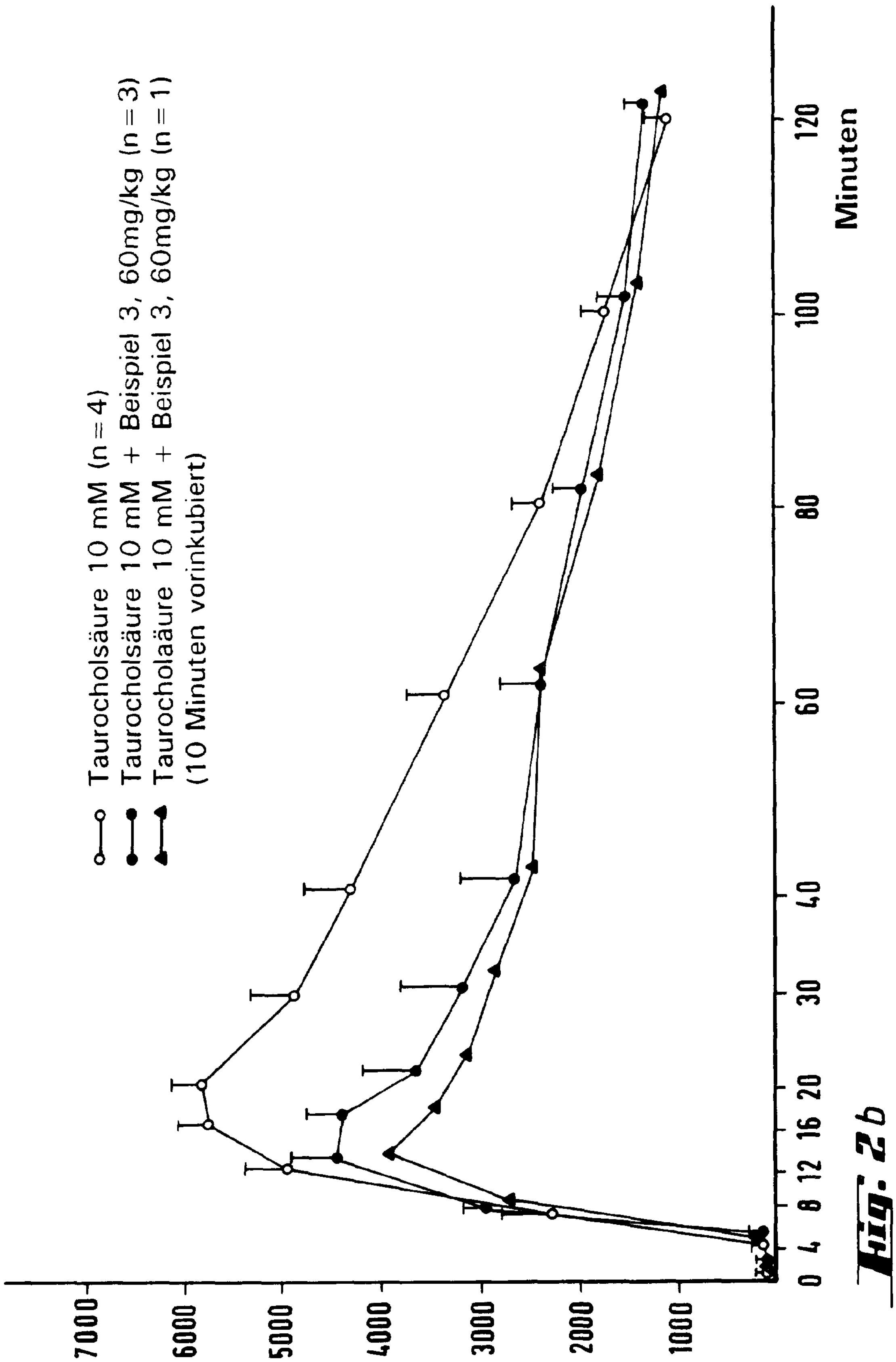
Taurocholsäure 10 mM (n = 4)
Taurocholsäure 10 mM + Beispiel 3, 60mg/kg (n = 3)
Taurocholaäure 10 mM + Beispiel 3, 60mg/kg (n = 1)
(10 Minuten vorinkubiert)
dpm je mg Galle
7000
6000
5000
4000
3000
2000
1000
0
4
8
12
16
20
30
40
60
80
100
120
Minuten

Fig. 2b

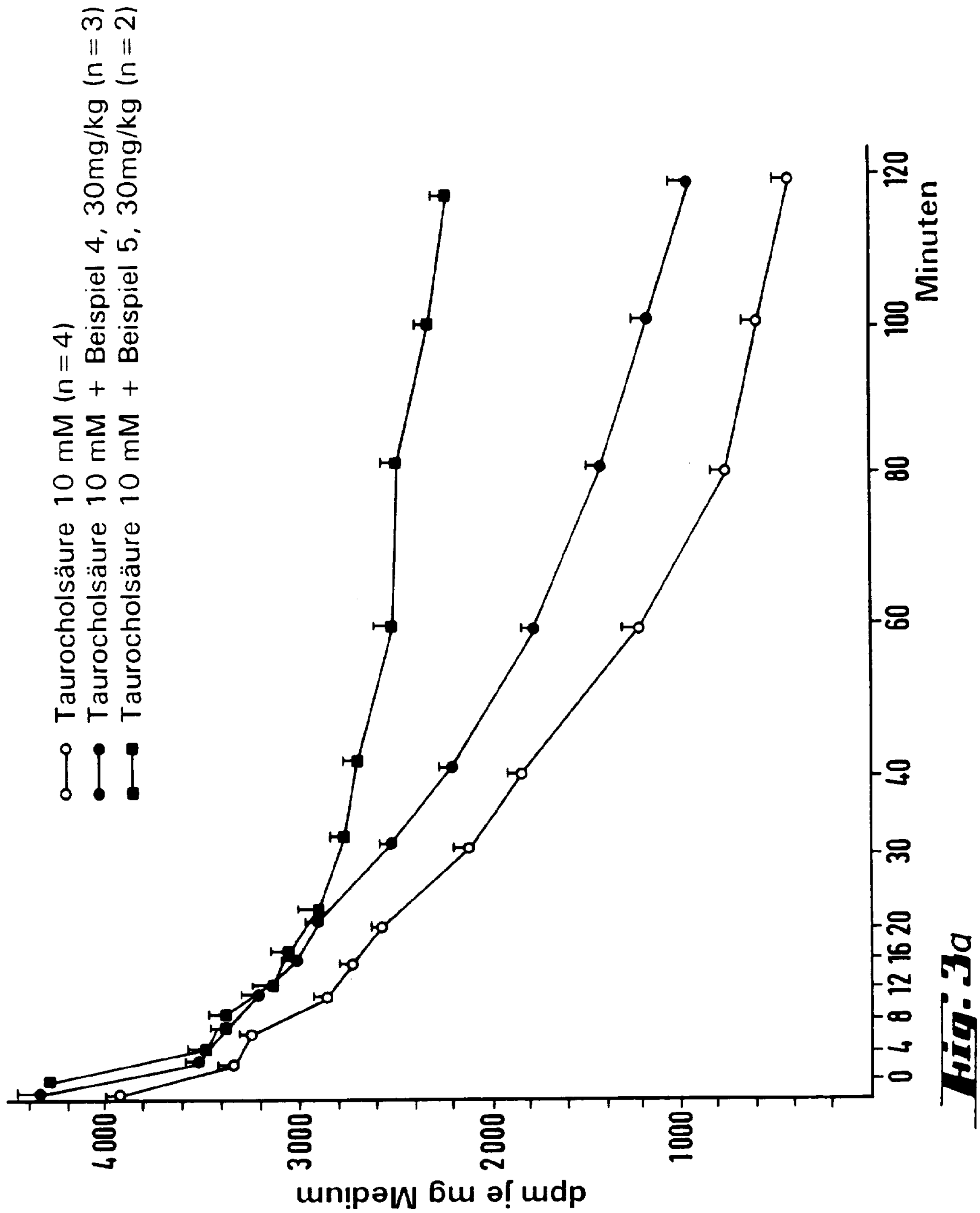

Taurocholsäure 10 mM (n = 4)
Taurocholsäure 10 mM + Beispiel 4, 30mg/kg (n = 3)
Taurocholsäure 10 mM + Beispiel 5, 30mg/kg (n = 2)
dpm je mg Medium
4000
3000
2000
1000
0 4 8 12 16 20
30
40
60
80
100
120
Minuten


Fig. 3a

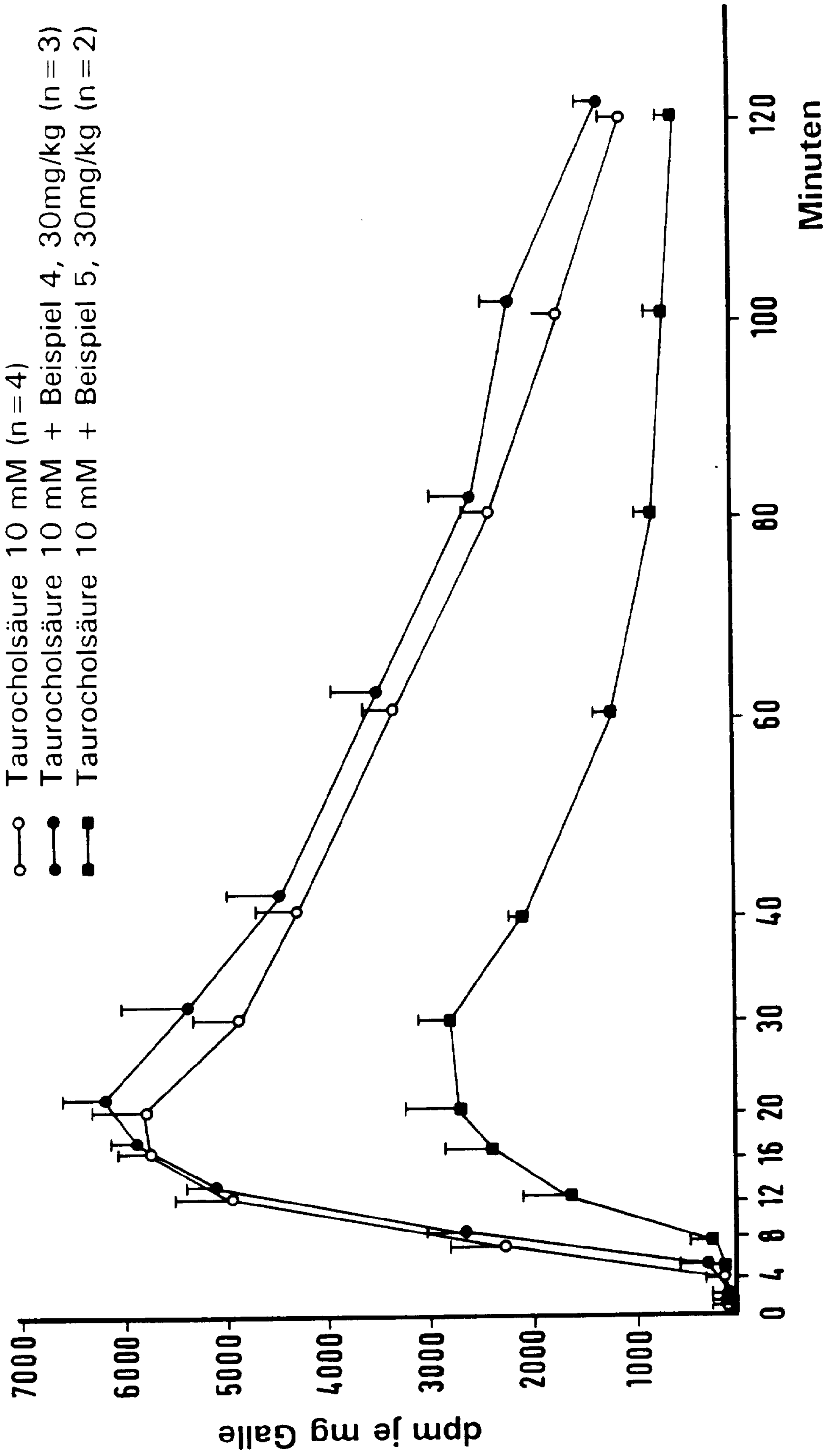

Taurocholsäure 10 mM (n = 4)
Taurocholsäure 10 mM + Beispiel 4, 30mg/kg (n = 3)
Taurocholsäure 10 mM + Beispiel 5, 30mg/kg (n = 2)
dpm je mg Galle
7000
6000
5000
4000
3000
2000
1000
0
4
8
12
16
20
30
40
60
80
100
120
Minuten


Fig. 3b

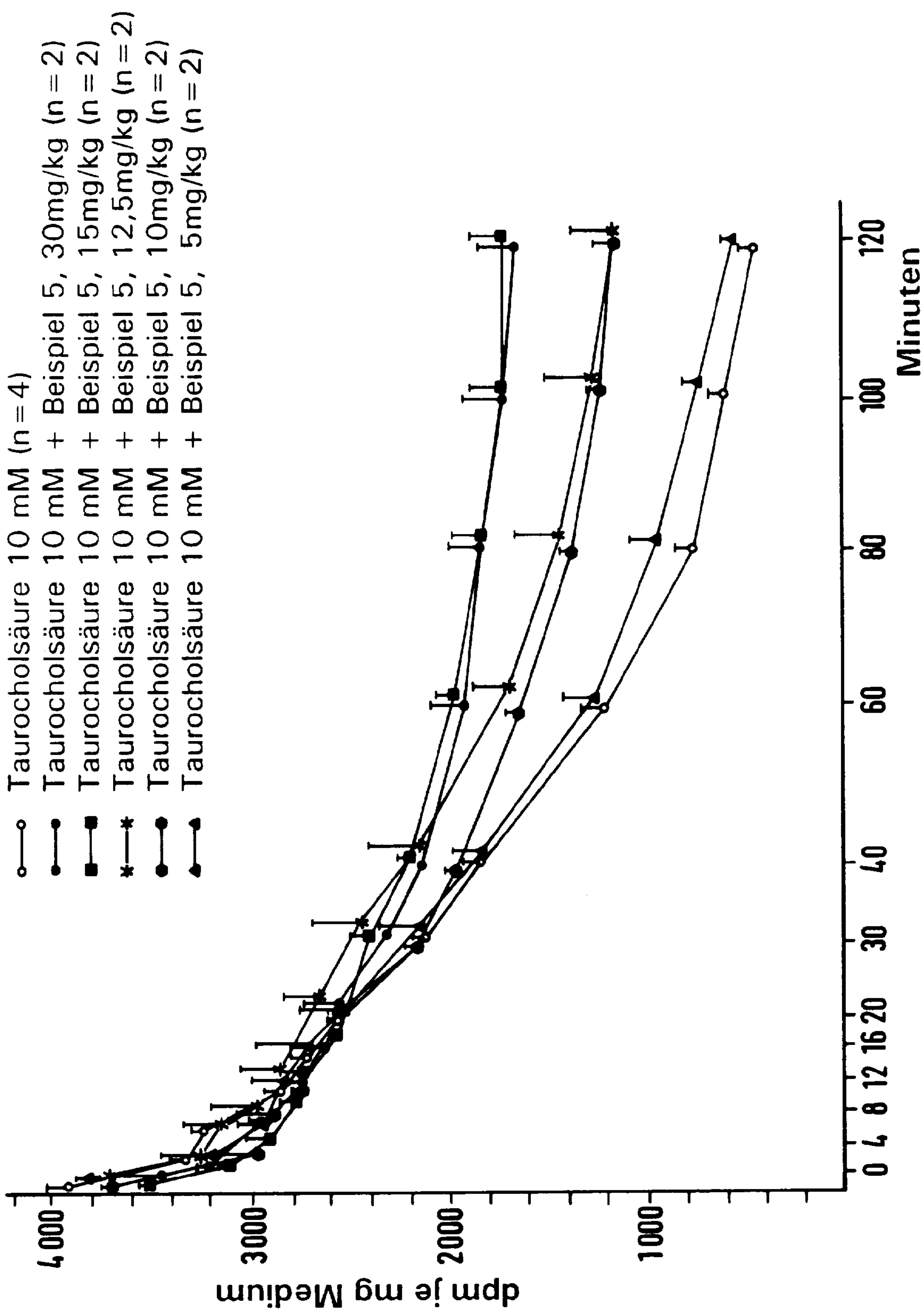
Taurocholsäure 10 mM (n = 4)
Taurocholsäure 10 mM + Beispiel 5, 30mg/kg (n = 2)
Taurocholsäure 10 mM + Beispiel 5, 15mg/kg (n = 2)
Taurocholsäure 10 mM + Beispiel 5, 12,5mg/kg (n = 2)
Taurocholsäure 10 mM + Beispiel 5, 10mg/kg (n = 2)
Taurocholsäure 10 mM + Beispiel 5, 5mg/kg (n = 2)
dpm je mg Medium
4000
3000
2000
1000
0 4 8 12 16 20 30 40 60 80 100 120
Minuten

Fig. 4a

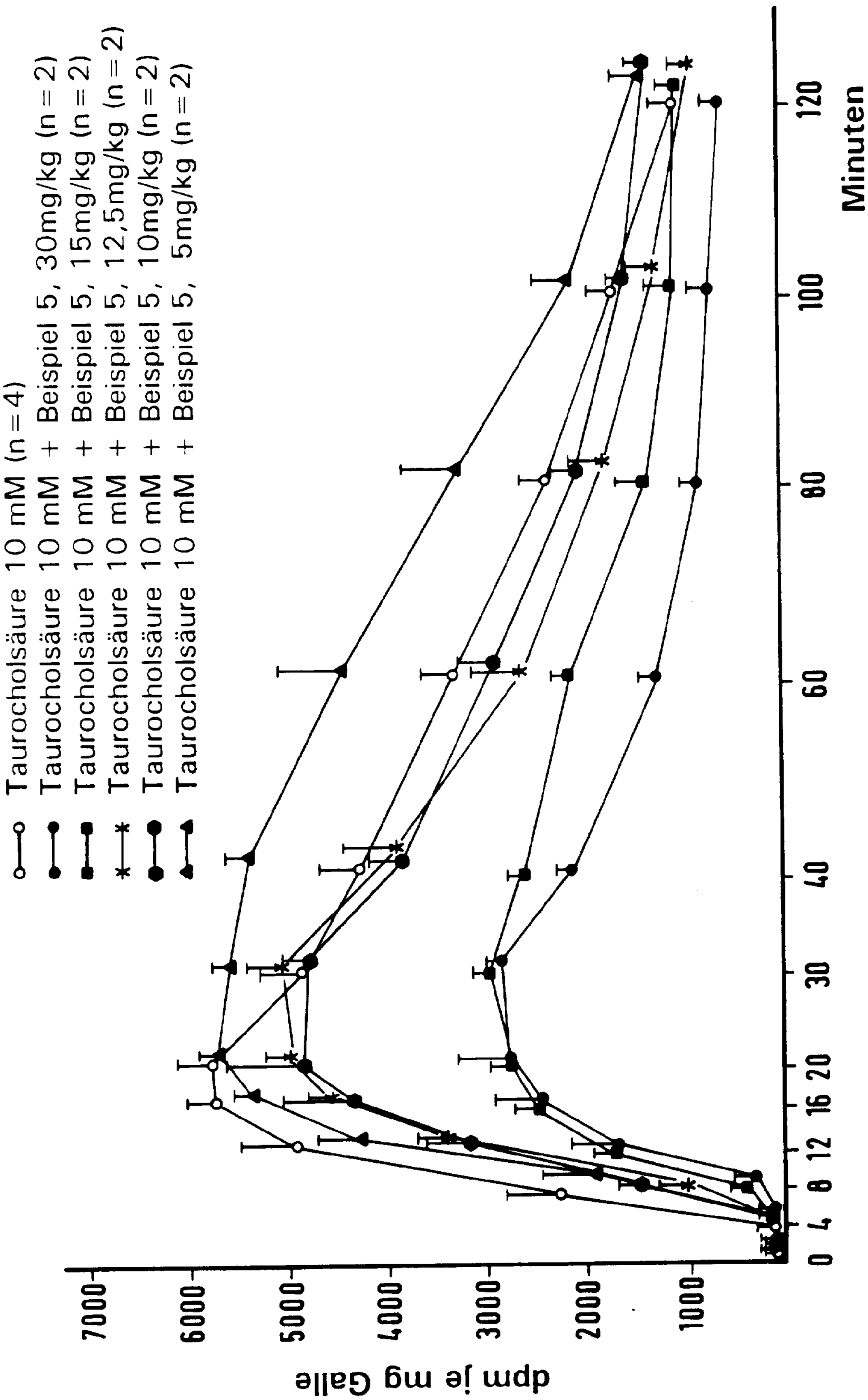
Taurocholsäure 10 mM (n = 4)
Taurocholsäure 10 mM + Beispiel 5, 30mg/kg (n = 2)
Taurocholsäure 10 mM + Beispiel 5, 15mg/kg (n = 2)
Taurocholsäure 10 mM + Beispiel 5, 12,5mg/kg (n = 2)
Taurocholsäure 10 mM + Beispiel 5, 10mg/kg (n = 2)
Taurocholsäure 10 mM + Beispiel 5, 5mg/kg (n = 2)
dpm je mg Galle
7000
6000
5000
4000
3000
2000
1000
0
4
8
12
16
20
30
40
60
80
100
120
Minuten

Fig. 4b

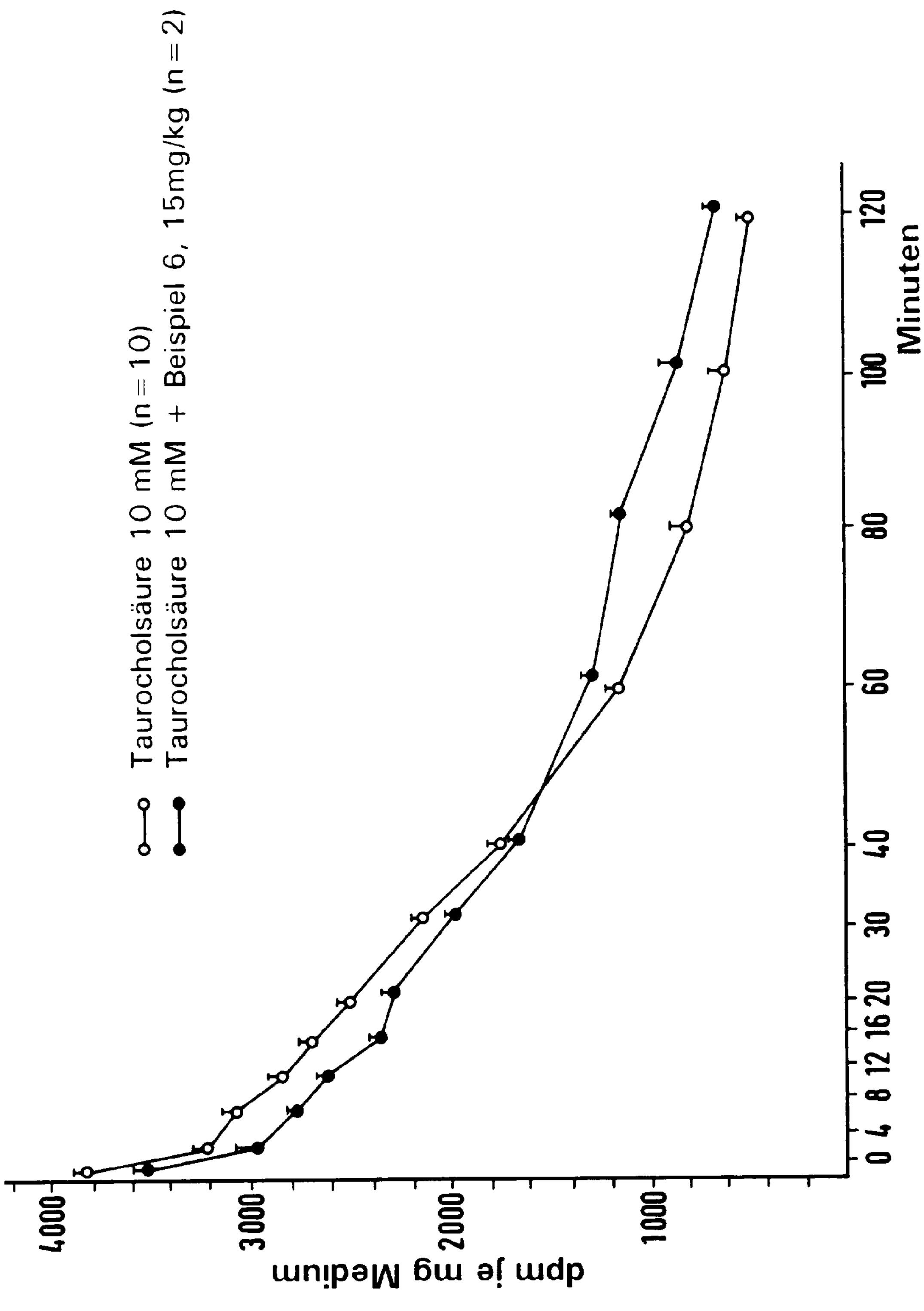
Taurocholsäure 10 mM (n = 10)
Taurocholsäure 10 mM + Beispiel 6, 15mg/kg (n = 2)
dpm je mg Medium
4000
3000
2000
1000
0 4 8 12 16 20
30
40
60
80
100
120
Minuten

Fig. 5a

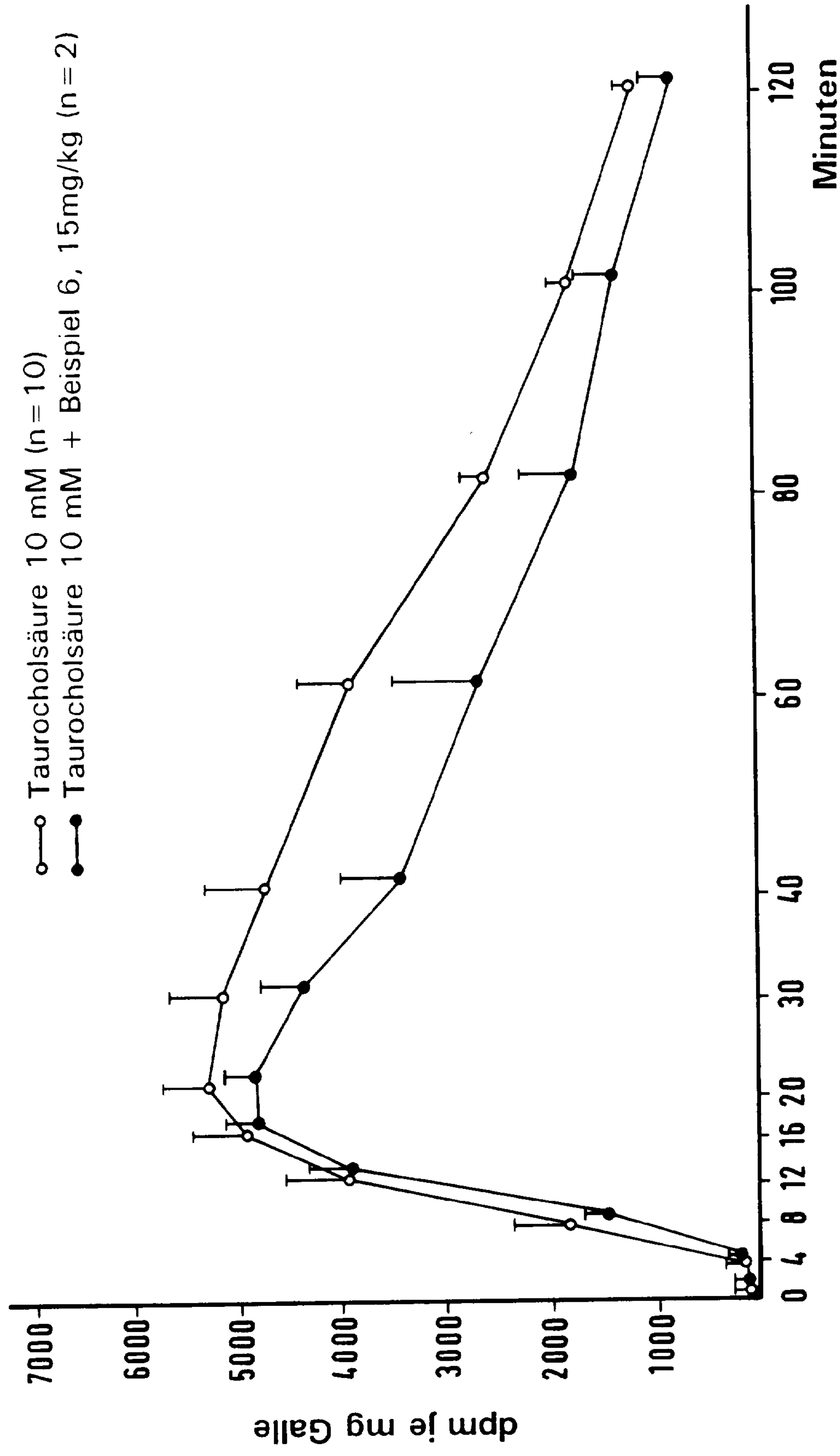
Taurocholsäure 10 mM (n = 10)
Taurocholsäure 10 mM + Beispiel 6, 15mg/kg (n = 2)
dpm je mg Galle
7000
6000
5000
4000
3000
2000
1000
0
4
8
12
16
20
30
40
60
80
100
120
Minuten

Fig. 5b

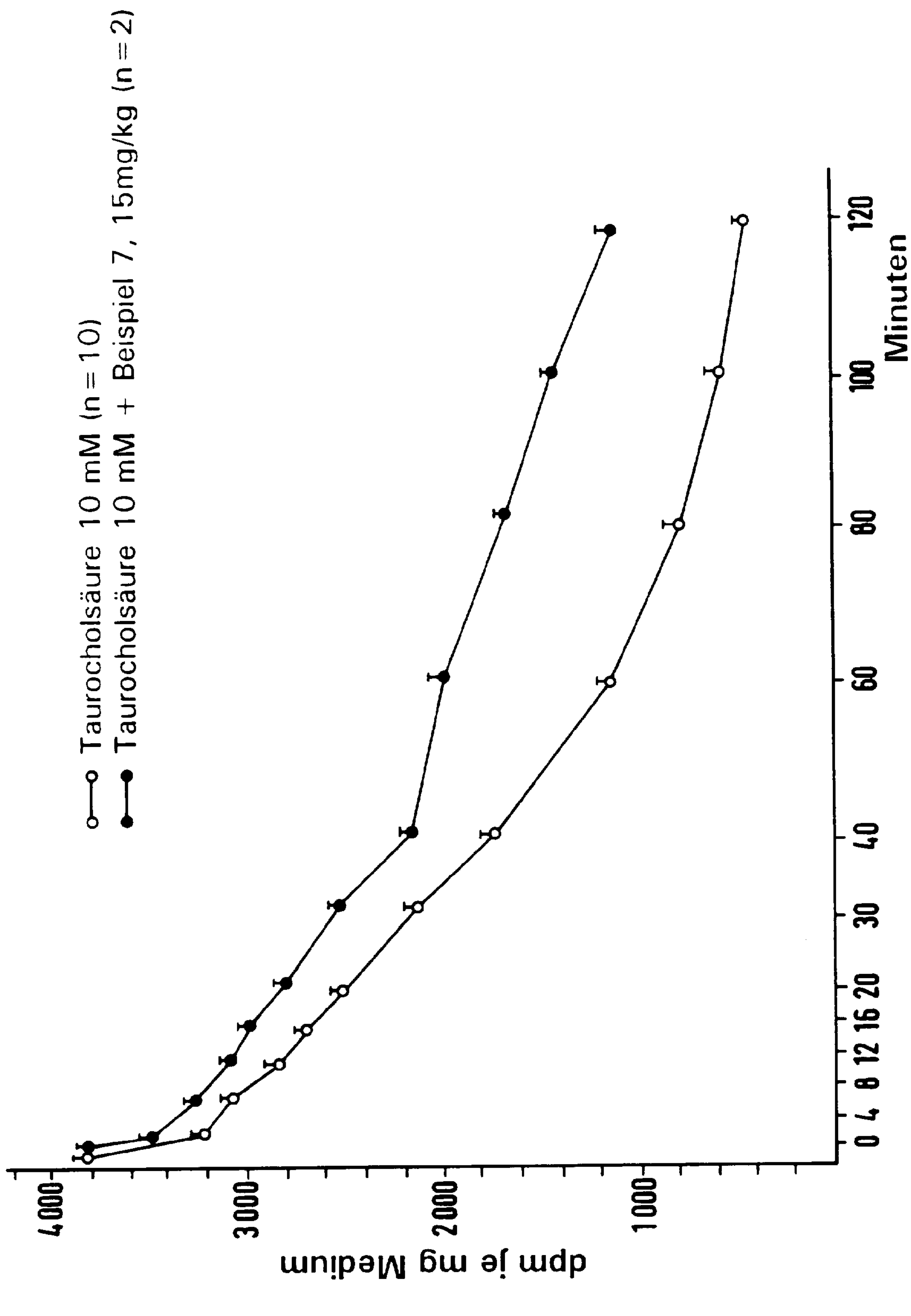
Taurocholsäure 10 mM (n = 10)
Taurocholsäure 10 mM + Beispiel 7, 15mg/kg (n = 2)
dpm je mg Medium
4000
3000
2000
1000
0 4 8 12 16 20
30
40
60
80
100
120
Minuten

Fig. 6a

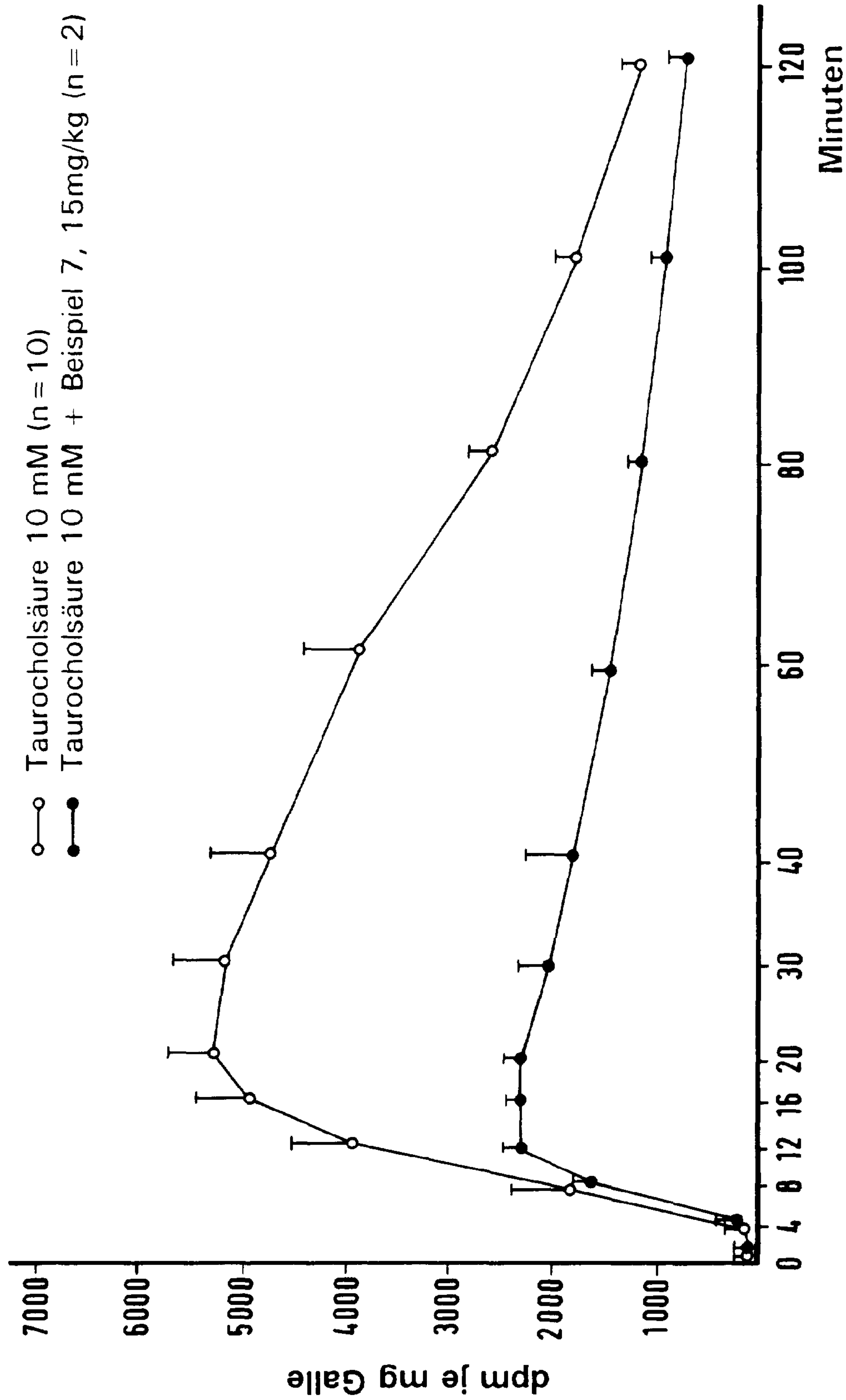
Taurocholsäure 10 mM (n = 10)
Taurocholsäure 10 mM + Beispiel 7, 15mg/kg (n = 2)
dpm je mg Galle
7000
6000
5000
4000
3000
2000
1000
0
4
8
12
16
20
30
40
60
80
100
120
Minuten

Fig. 6b

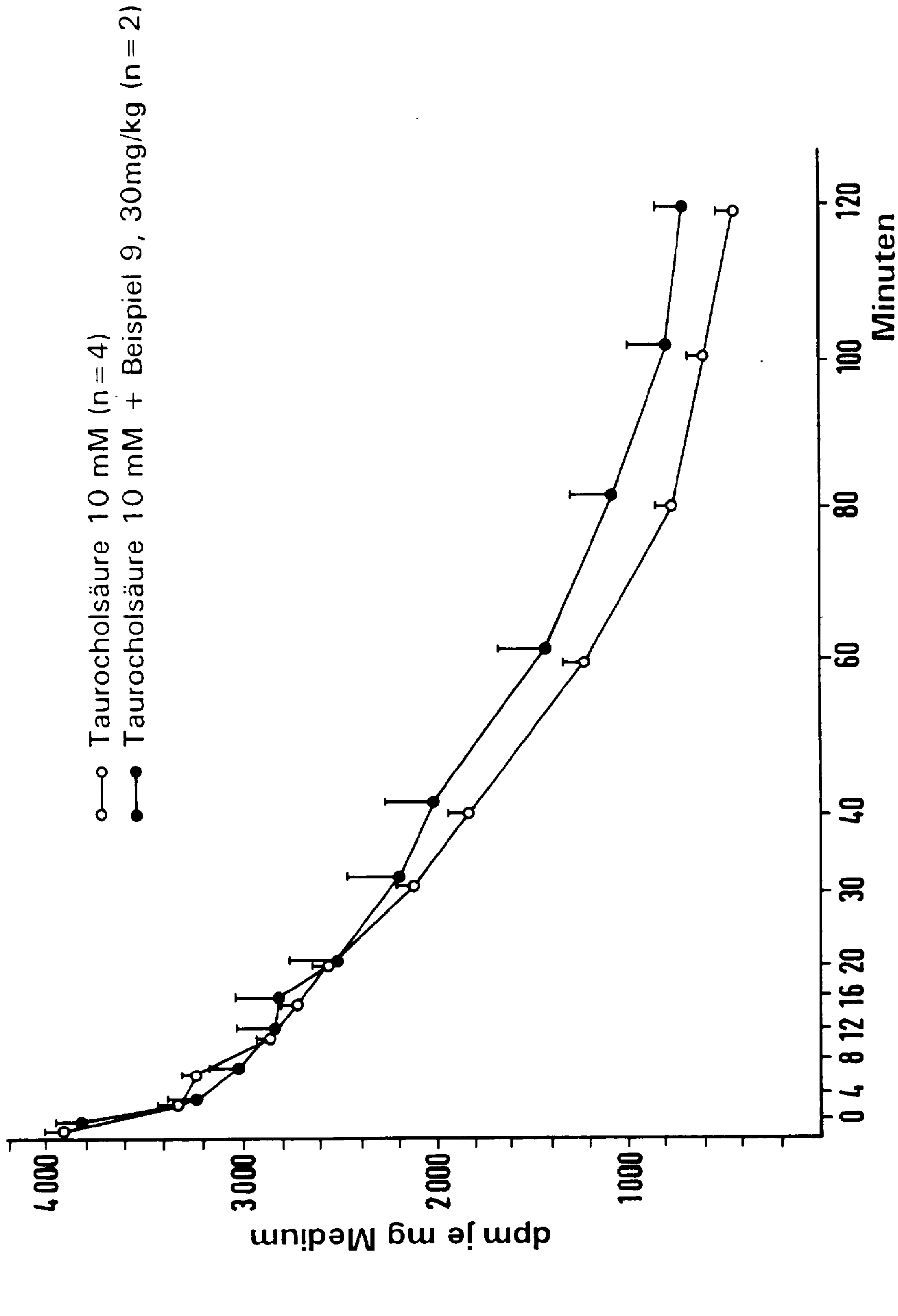

Taurocholsäure 10 mM (n = 4)
Taurocholsäure 10 mM + Beispiel 9, 30mg/kg (n = 2)
dpm je mg Medium
4 000
3 000
2 000
1000
0 4 8 12 16 20 30 40 60 80 100 120
Minuten


Fig. 7a

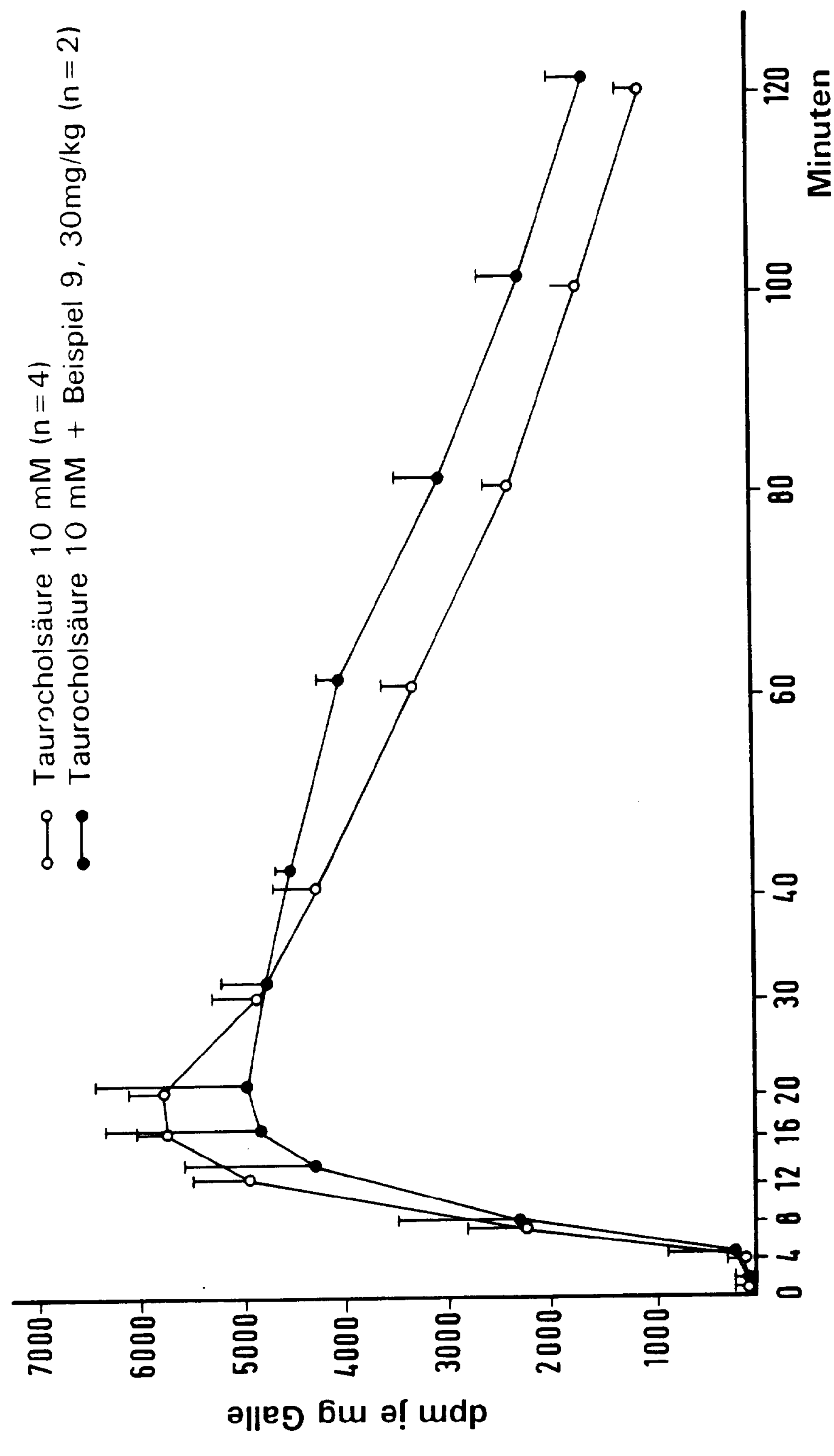
Taurocholsäure 10 mM (n = 4)
Taurocholsäure 10 mM + Beispiel 9, 30mg/kg (n = 2)
dpm je mg Galle
7000
6000
5000
4000
3000
2000
1000
0
4
8
12
16
20
30
40
60
80
100
120
Minuten

Fig. 1b

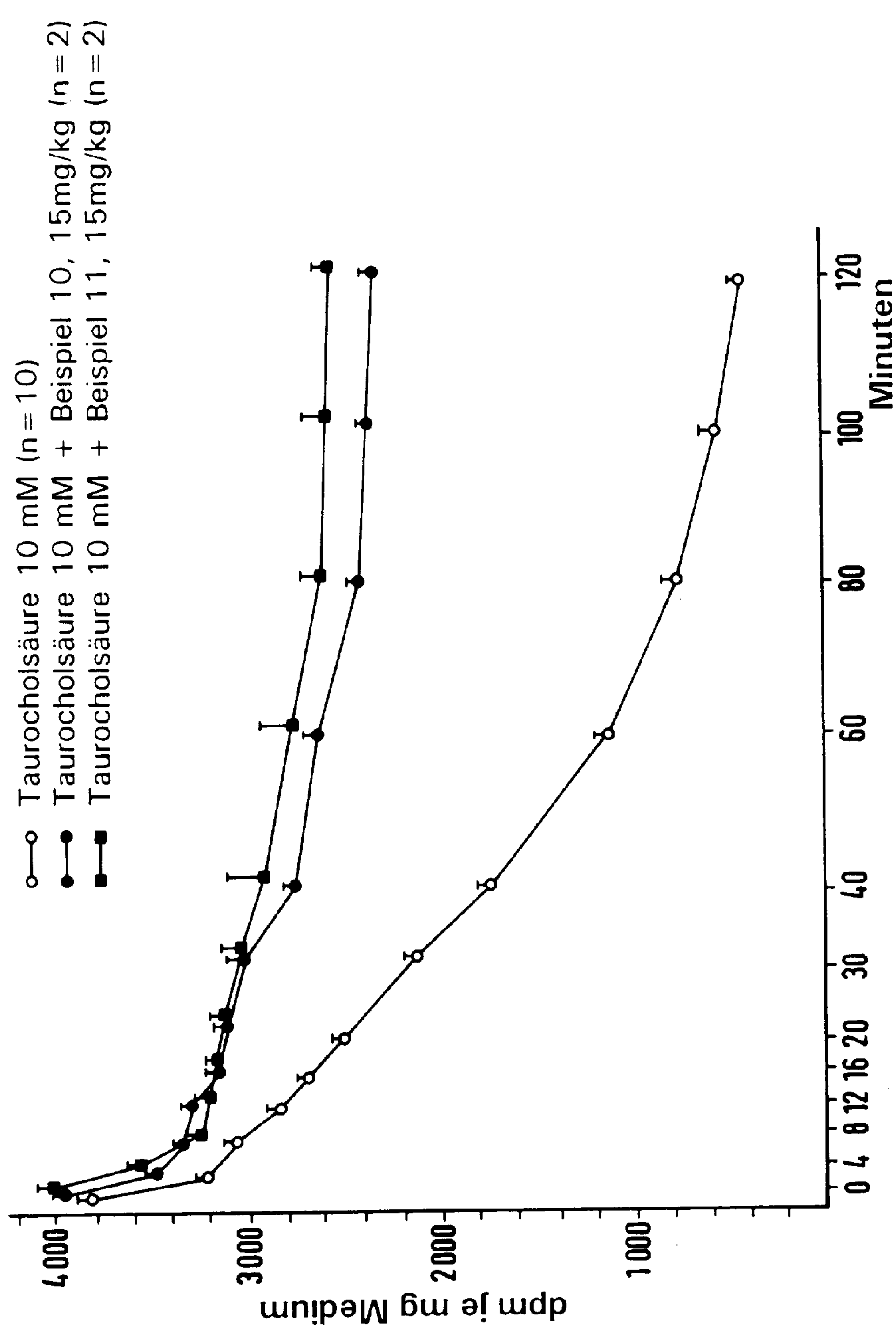
Taurocholsäure 10 mM (n = 10)
Taurocholsäure 10 mM + Beispiel 10, 15mg/kg (n = 2)
Taurocholsäure 10 mM + Beispiel 11, 15mg/kg (n = 2)
4000
3000
2000
1000
dpm je mg Medium
0 4 8 12 16 20
30
40
60
80
100
120
Minuten

Fig. 8a

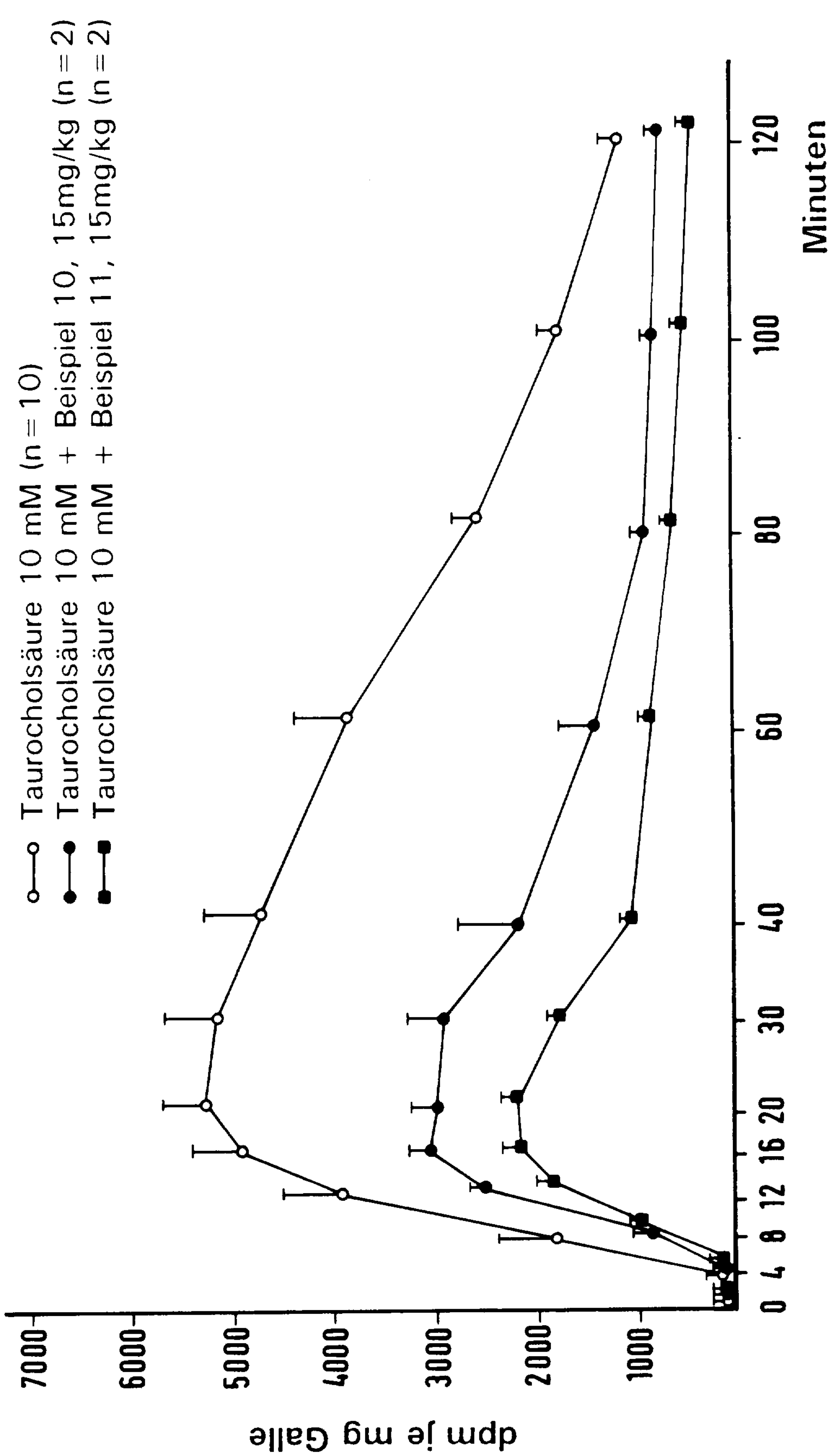
Taurocholsäure 10 mM (n = 10)
Taurocholsäure 10 mM + Beispiel 10, 15mg/kg (n = 2)
Taurocholsäure 10 mM + Beispiel 11, 15mg/kg (n = 2)
dpm je mg Galle
7000
6000
5000
4000
3000
2000
1000
0
4
8
12
16
20
30
40
60
80
100
120
Minuten

Fig. 8b

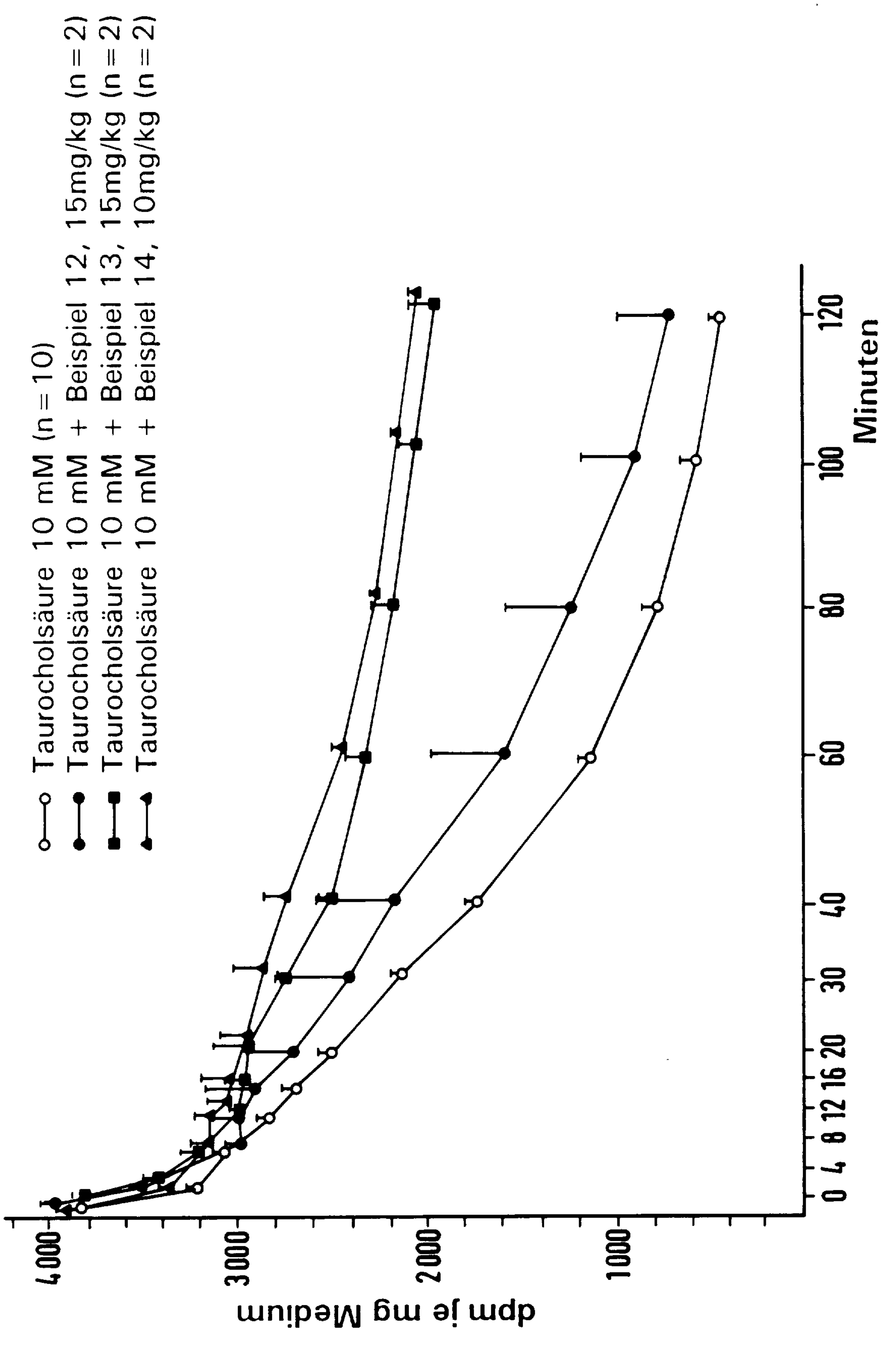
Taurocholsäure 10 mM (n = 10)
Taurocholsäure 10 mM + Beispiel 12, 15mg/kg (n = 2)
Taurocholsäure 10 mM + Beispiel 13, 15mg/kg (n = 2)
Taurocholsäure 10 mM + Beispiel 14, 10mg/kg (n = 2)
dpm je mg Medium
4000
3000
2000
1000
0 4 8 12 16 20
30
40
60
80
100
120
Minuten

Fig. 9a

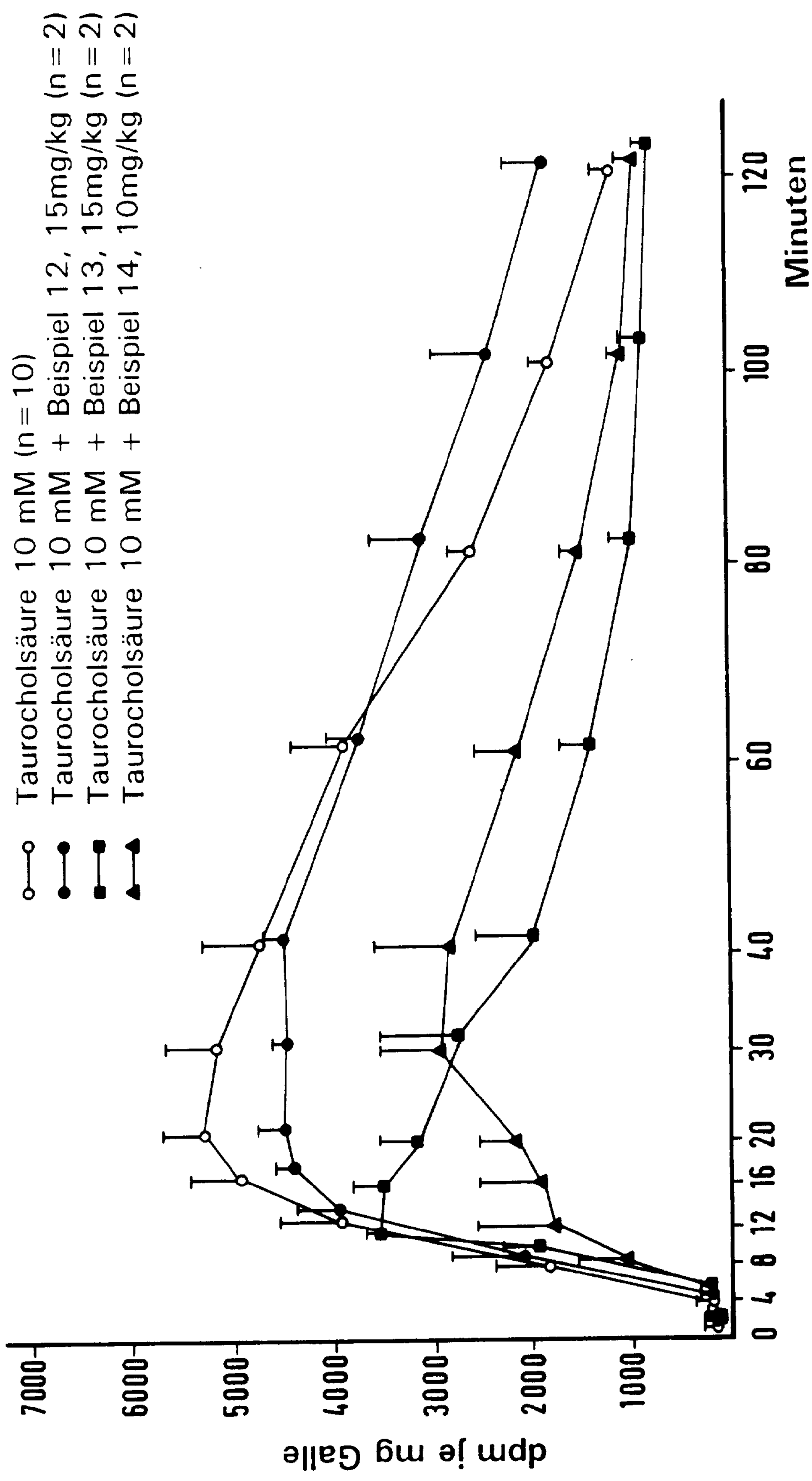
Taurocholsäure 10 mM (n = 10)
Taurocholsäure 10 mM + Beispiel 12, 15mg/kg (n = 2)
Taurocholsäure 10 mM + Beispiel 13, 15mg/kg (n = 2)
Taurocholsäure 10 mM + Beispiel 14, 10mg/kg (n = 2)
dpm je mg Galle
7000
6000
5000
4000
3000
2000
1000
0
4
8
12
16
20
30
40
60
80
100
120
Minuten

Fig. 9b

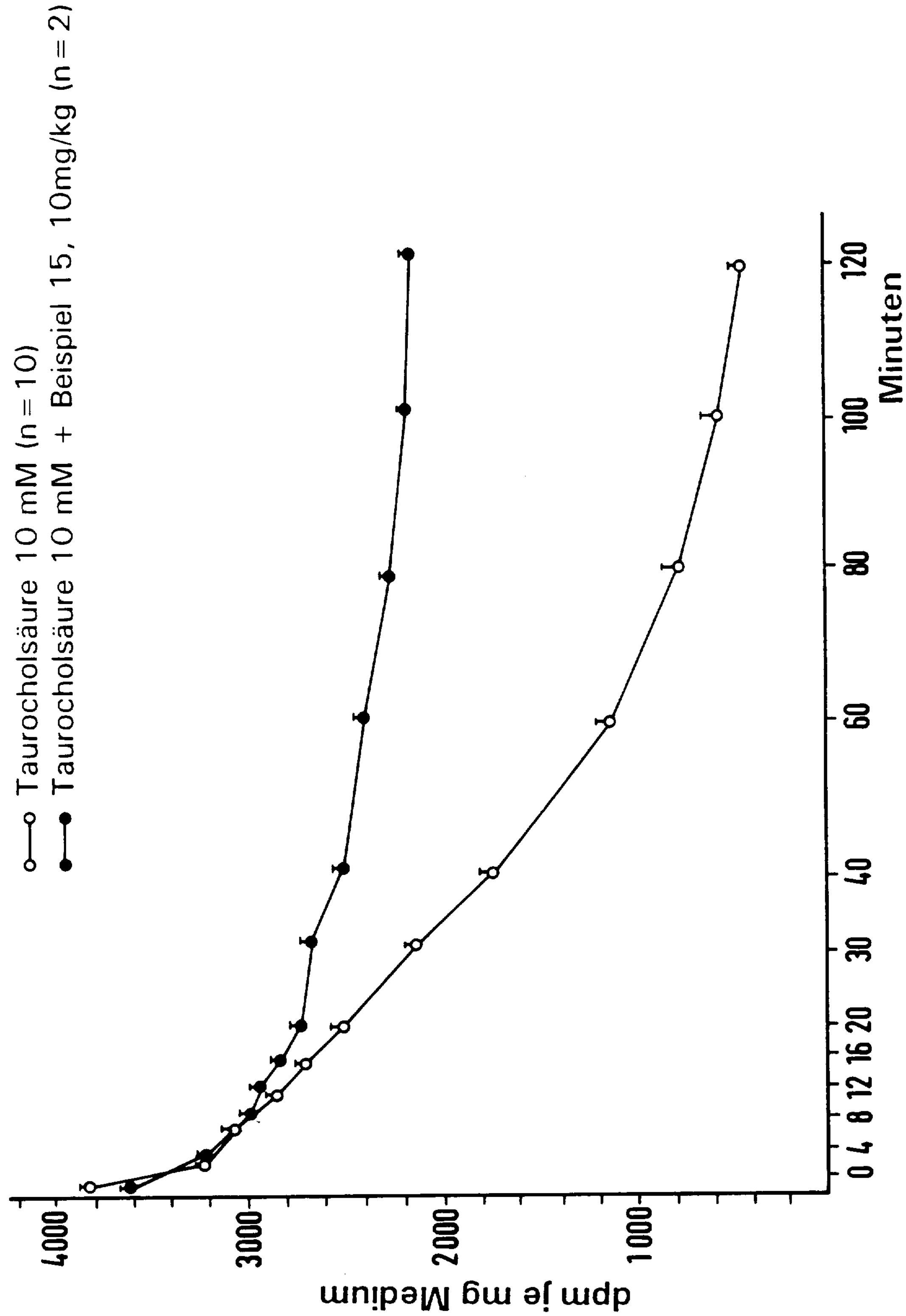
Taurocholsäure 10 mM (n = 10)
Taurocholsäure 10 mM + Beispiel 15, 10mg/kg (n = 2)
dpm je mg Medium
4000
3000
2000
1000
0 4 8 12 16 20
30
40
60
80
100
120
Minuten

Fig. 10a

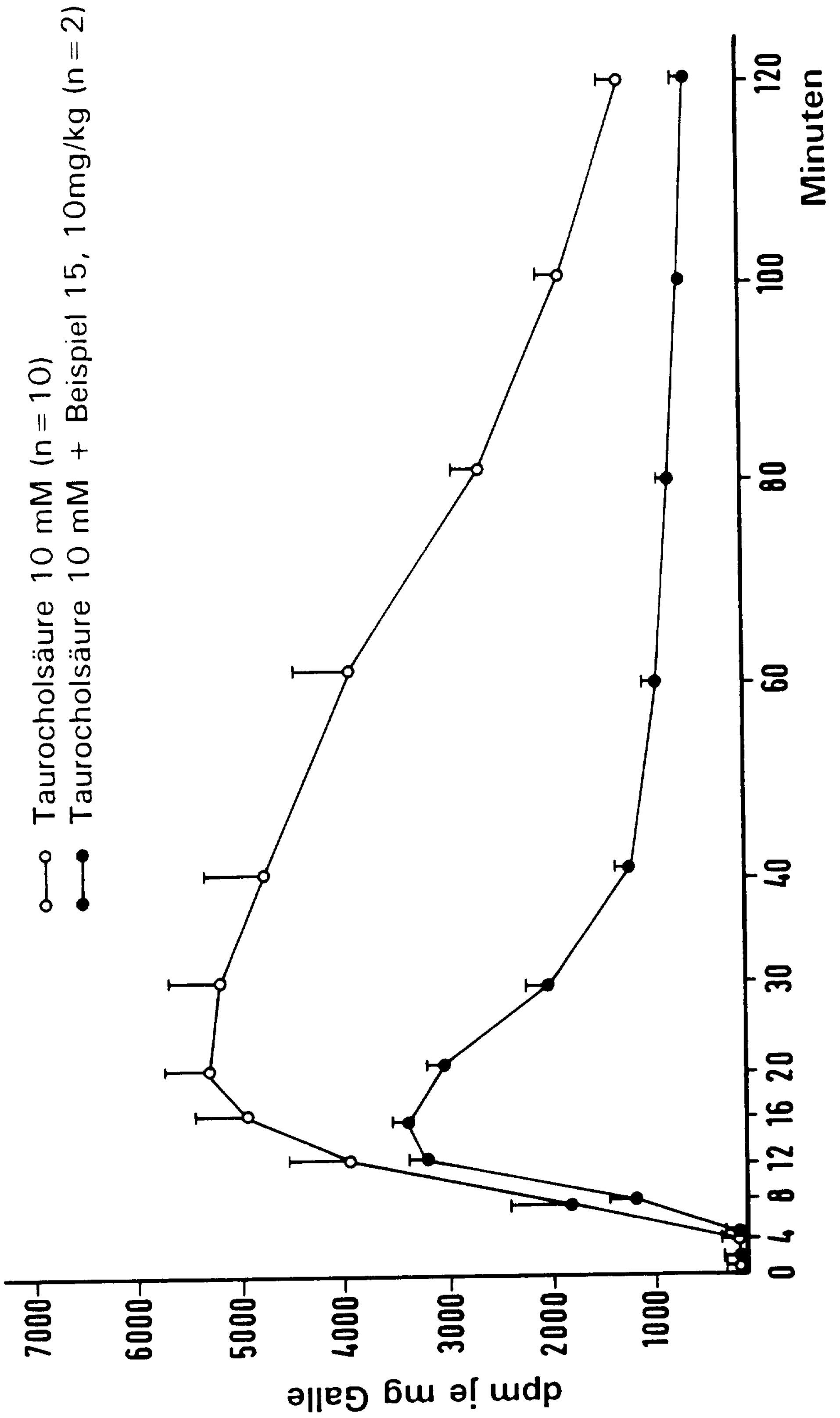
Taurocholsäure 10 mM (n = 10)
Taurocholsäure 10 mM + Beispiel 15, 10mg/kg (n = 2)
dpm je mg Galle
7000
6000
5000
4000
3000
2000
1000
0 4 8 12 16 20 30 40 60 80 100 120
Minuten

Fig. 10b

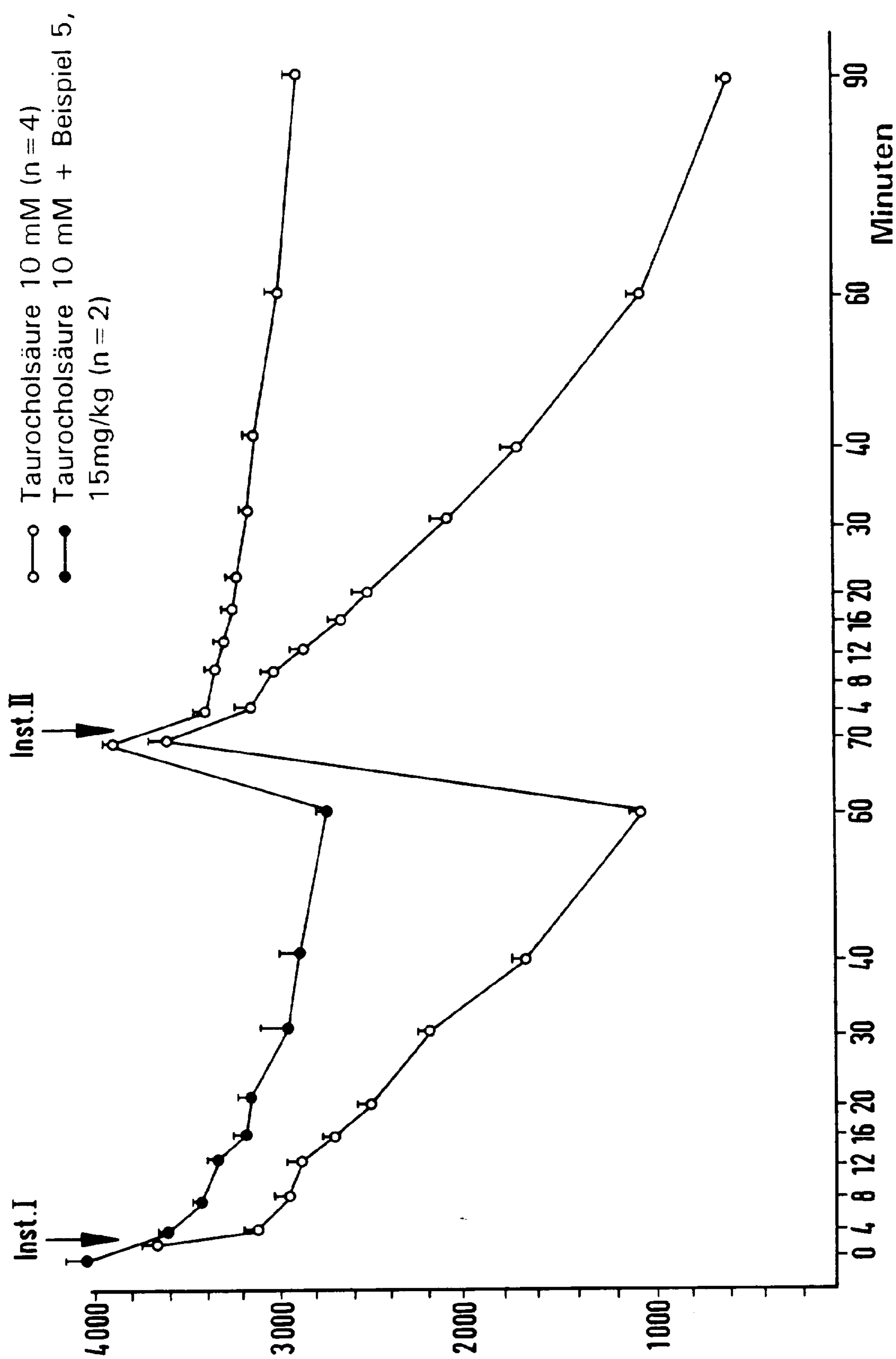
Taurocholsäure 10 mM (n = 4)
Taurocholsäure 10 mM + Beispiel 5,
15mg/kg (n = 2)
Inst.I
Inst.II
4000
3000
2000
1000
dpm je mg Medium
0 4 8 12 16 20
30
40
60
70 4 8 12 16 20
30
40
60
90
Minuten

Fig. 11a

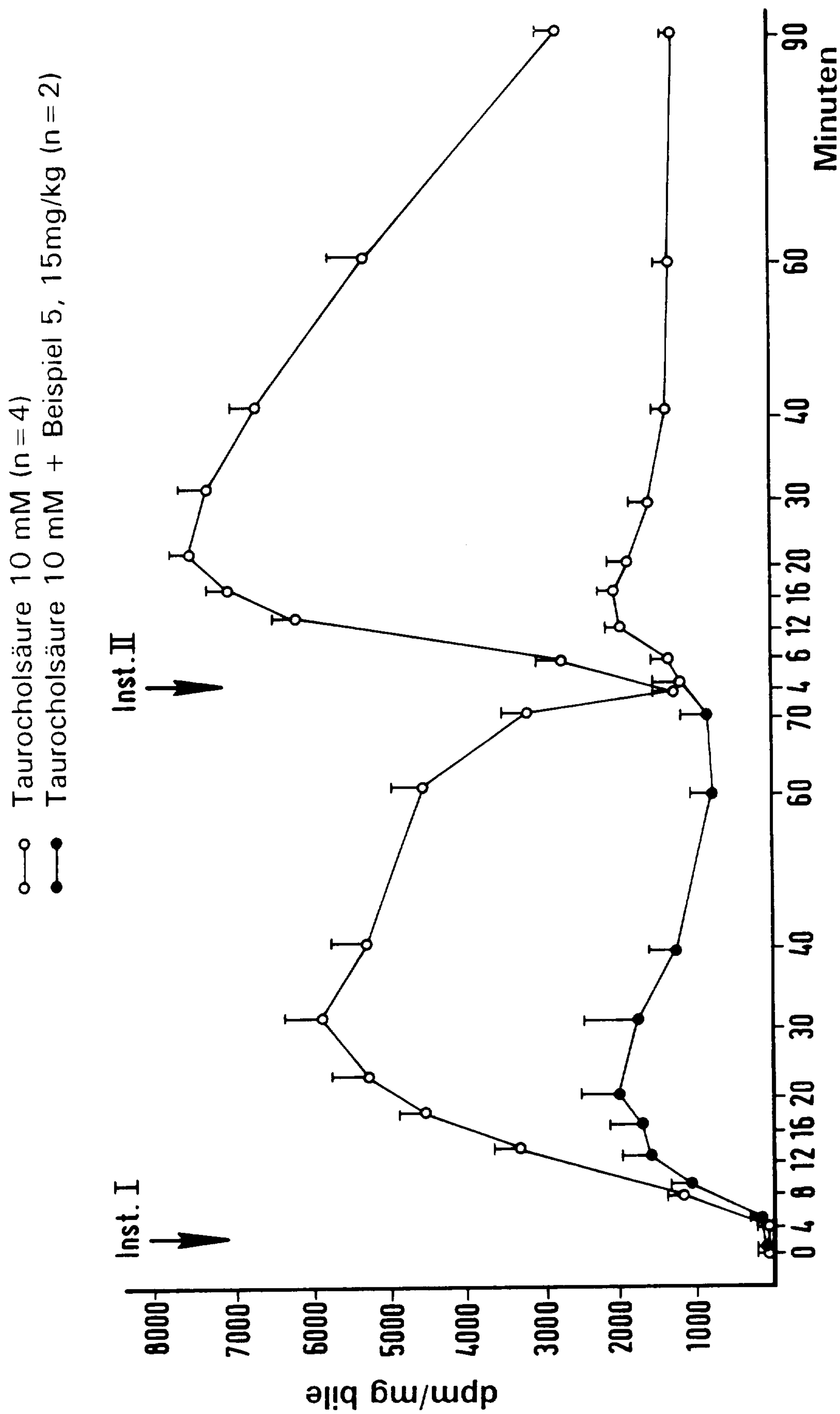
Taurocholsäure 10 mM (n = 4)
Taurocholsäure 10 mM + Beispiel 5, 15mg/kg (n = 2)
Inst. I
Inst. II
dpm/mg bile
8000
7000
6000
5000
4000
3000
2000
1000
0 4 8 12 16 20 30 40 60 70 4 6 12 16 20 30 40 60 90
Minuten

Fig. 11b